Europäisches Patentamt

European Patent Office

Office européen des brevets

(11) Veröffentlichungsnummer: **0 260 484 B1**

# EUROPÄISCHE PATENTSCHRIFT

(45) Veröffentlichungstag der Patentschrift: **03.04.91**

(51) Int. Cl.⁵: **A61B 17/60**

(21) Anmeldenummer: **87112273.5**

(22) Anmeldetag: **25.08.87**

(54) **Externe Befestigungsvorrichtung.**

(30) Priorität: **26.08.86 DE 3628972**

(43) Veröffentlichungstag der Anmeldung:
**23.03.88 Patentblatt 88/12**

(45) Bekanntmachung des Hinweises auf die
Patenterteilung:
**03.04.91 Patentblatt 91/14**

(84) Benannte Vertragsstaaten:
**AT BE CH DE ES FR GB GR IT LI LU NL SE**

(56) Entgegenhaltungen:
**FR-A- 2 405 063**
**GB-A- 2 086 231**
**US-A- 4 604 996**

**JOURNAL OF BONE AND JOINT SURGERY,
Band 63-A, Nr. 8, Oktober 1981, Seiten
1289-1291, Boston, Massachusetts, US; M.
PRITSCH et al.: "Manipulation and external
fixation of metacarpal fractures"**

(73) Patentinhaber: **Dunsch-Herzberg, Renate
Holmer Strasse 165
W-2000 Wedel/Holstein(DE)**

Patentinhaber: **Voss, Gudrun
Cranz 26
W-2081 Hetlingen(DE)**

(72) Erfinder: **Herzberg, Wolfgang, Dr. med.
Holmer Strasse 165
W-2000 Wedel/Holstein(DE)**

(74) Vertreter: **Richter, Werdermann & Gerbaulet
Neuer Wall 10
W-2000 Hamburg 36(DE)**

## Beschreibung

Die Erfindung betrifft einen Fixateur externe aus mindestens einem flexiblen schlauchförmigen Teil aus Kunststoff, der außerhalb des Patienten gegen-über den zu fixierenden Knochenfragmenten anordbar und der mit in den verschiedenen Knochenfragmenten befestigten Drähten, Nägeln oder Schrauben verbindbar ist, wobei der mit einem aushärtbaren Material gefüllte schlauchförmige, Teil nach dem Aushärten des Materials mit den Drähten, Nägeln oder Schrauben ein festes Gestell bildet, das die Knochenfragmente zum Zusammenwachsen in einer gewünschten Stellung zusammenhält.

Fixateur externe dienen zur orthopädischen Fixierung und zum Zusammenhalten von Knochenfragmenten. Mit einem Fixateur externe werden gebrochene Knochen in ihrer gegenseitigen Lage fixiert, damit die Heilung in der richtigen Position erfolgen kann. Dazu werden in den Knochen hinein oder durch den Knochen hindurch Knochennägel, vorzugsweise mit Gewinde, z.B. Schanz'sche Schrauben oder Brückenstäbe, eingesetzt und an ihren aus dem Körper herausragenden Enden durch wenigstens ein stabförmiges Element, z .B. einen Gleitstab oder einen Führungsstab, miteinander verbunden. Diese Verbindung erfolgt durch Spannelemente, die als Klemmstücke, Gelenkstükke und ggfs. als Kugelgelenk, als Kupplung oder Griffstücke od.dgl. ausgebildet sind. So kann eine Rahmenkonstruktion gebildet werden, mit der die Knochen-Teile stabil fixiert und gehalten wer den.

Bekannte Spannelemente bestehen aus nichtoxidierendem Stahl. Beim Anlegen des Fixateurs und Spannen der Stäbe ist dabei das gesamte Rahmengebilde relativ locker, wackelig und in sich verschiebbar, da die gegenseitig zu haltenden Stäbe in den Spannelementen gelöst sein müssen, um sie in die richtige Position zu bringen. So ist das Anlegen eines Fixateurs letztlich nicht von einer einzigen Person vornehmbar, sondern es werden weitere Hilfskräfte erforderlich, die die Gelenke und Doppelgelenke und den Rahmen halten müssen, bis nach der Positionierung schließlich das Spannen und Fixieren vorgenommen werden kann. Um diese Arbeit zu erleichtern, können gesonderte Spanngeräte angewendet werden. Da Stahl einen sehr hohen Elastizitätskoeffizienten und eine glatte Oberfläche aufweist, ist es nicht möglich, durch Anziehen einer Spannschraube nur eine schwache Klemmung zu erreichen; wegen des hohen Elastizitätskoeffizienten bewirkt schon eine geringe Drehung einer Feststellschraube eine sehr hohe Änderung des Klemmdrukkes. Beim Anpassen des Fixateurs können daher die Stäbe, Knochen-Nägel oder -Schrauben nur lose in den Spannelementen liegen. Hinzu kommt noch, daß die für den Fixateur verwendeten Teile und Elemente aufgrund ihrer Werkstoffauswahl Röntgenaufnahmen beeinträchtigen. Außerdem sind die Herstellungskosten für derart aus-gebildete Fixateure sehr hoch.

Vorherrschend ist die Verwendung von Metallbauteilen, aus denen der Fixateur je nach individuellen Erfordernissen vom Chirurgen zusammengesetzt wird. Um eine Röntgentransparenz bei den Fixateuren zu erhalten, werden auch Bauteile aus metallfreien Werkstoffen verwendet. So ist durch die DE-A-27 45 504 eine Vorrichtung zur Ruhigstellung und/oder Stützung von Gliedmaßen von Menschen und Tieren, welche paarweise gegenüberliegende und mindestens jeweils eine Knochenschraube oder einen Knochennagel aufnehmende Verbindungsteile aufweist, die über Klemmelemente mit Verbindungs- bzw. Gewindestäben in Verbindung stehen, bekannt . Die Verbindungsteile, die Klemmelemente und die Verbindungsstäbe sowie die integrierten Spannschlösser bestehen dabei aus faserverstärkten thermoplastischen Kunststoffen.

Des weiteren ist durch die DE-A-31 41 909 eine orthopädische Bruchfixierungsvorrichtung mit mindestens einem außerhalb des Patienten liegenden länglichen Teil, bekannt, der mit Knochenstiften od.dgl. verbunden ist, die in den unterschiedlichen Bruchstücken des gebrochenen Knochens befestigt sind, wobei der längliche Teil und die Stifte ein festes Gestell bilden, das die Bruchstücke in der gewünschten Stellung zum Zusammenwachsen hält. Der außerhalb des Patienten liegende längliche Teil, der mit Knochenstiften mit den unterschiedlichen Bruchstükken des gebrochenen Knochens befestigt ist, umfaßt einen länglichen Träger flexibler Form, der mit einem härtbaren Material gefüllt ist, das im Träger in einem inaktiven fließfähigen Zustand vorliegt.

Bei diesem Fixateur externe wird das aushärtbare Material vor der Montage des Schlauchsystems und der Fixierung der Knochenfragmente in den Innenraum des geschlossenen Schlauchsystems eingegeben. Verwendet werden aushärtbare Materialien, bei denen die Aushärtwirkung bis zum Zeitpunkt des Bedarfs verzögerbar ist. Insbesondere werden solche aushärtbaren Materialien eingesetzt, die sich unter der Einwirkung von sichtbarem oder anderem Licht aushärten, wobei der mit dem aushärtbaren Material gefüllte schlauchförmige Teil mit einer lichtundurchlässigen Außenabdeckung umhüllt ist. die für den Aushärtprozeß entfernt wird. Als Kunststoffschlauch wird ein wellenförmig ausgebildeter Schlauch verwendet.Durch die Verwendung eines derart ausgebildeten Schlauches kann es zu Querschnittsveränderungen kommen, wenn der Schlauch in verschiedenen Ebenen verlegt und dabei abgeknickt werden muß.

Durch die FR-A-2 405 063 ist ein Fixateur externe bekannt, bei dem von vorgeformten und festen Bau-teilen ausgegangen wird. Die einzelnen Bauteile be-stehen aus mit Glasfasern verstärktem Kunststoff. Fixaeure externe, bei denen vorgeformte und feste Bauteile verwendet werden, sind in der Herstellung der jeweils erforderlichen Bausätze sehr kostspielig. Ein bestimmter Bausatz be-sitzt nur eine auf bestimmte Frakturen beschränkte Anwendbarkeit, was die Vorratshaltung und Lagerung eines umfangreichen Sortiments der einzelnen Bauteile zur Folge hat. Gewindetragende Bauteile weisen bei wiederholter Verwendung Verschleißerscheinungen auf. Sofern metallische Werkstoffe zur Anwendung kommen, ist die röntgenologische Kontrolle der Frakturheilung unter Umständen erheblich behindert. Mit jedem versorgten Patienten ist ein Teil des vorrätigen Bausatzes über Wochen blokkiert, was eine weitere Asdehnung einer Vorratshaltung der einzelnen Bauteile notwendig macht. Der unzuverlässige und der durchreisende Patient kehrt an das primär versorgende Krankenhaus nicht zurück. Die eingesetzten Bauteile gelangen nicht wieder in den Besitz des Krankenhauses zurück, wodurch ein hoher Verlust an Bauteilen entsteht. Die starren Bausysteme erzwingen darüber hinaus in unterschiedlichem Ausmaß eine koordinatengerechte Anordnung von Knochenfragmenten und Fixateur externe Um unter diesen Zwängen zu einem guten Operationsergebnis gelangen zu können, sind nicht selten ein hohes Maß an Erfahrung sowie räumlicher und planerischer Vorstellungskraft erforderlich.

Eine extern anzubringende Skelett-Fixierungseinrichtung ist durch die US-A-4,604,996 bekannt. Bei diesem Fixator handelt es sich um eine Knochenfragmente fixierende Einrichtung für Knochenbrüche bei Pferdebeinen, wobei mit dieser Einrichtung gleichzeitig eine abstützende Wirkung erzielt wird, um das verletzte Pferdebein ruhigzustellen und keiner Gewichtsbelastung auszusetzen. Diese Einrichtung besteht aus parallel zu beiden Seiten des zu behandelnden Pferdebeines angebrachten und parallel zueinander verlaufenden Stützstäben, die hufseitig in gabelförmige Abschnitte übergehen, die im Bereich der Hufsohle an einer Trägerplatte befestigt sind, die wiederum mit der Hufsohle vermittels Schraubverbindungen verbunden ist. Die Fixierung des gebrochenen Knochens erfolgt mittels Stiftschrauben z .B. Schanz'sche Schrauben, die durch den Knochen hindurchge-führt sind und deren Enden im Bereich der parallel zum Pferdebein verlaufenden Stützstäbe liegen. Um die Schrauben mit ihren Enden mit den Stützstäben fest verbinden zu können, sind zwei rohrförmige Formkörper vorgesehen, die einendseitig verschlossen ausgebildet sind und in deren Innenräumen die Stützstäbe zu liegen kommen. Die Innenräume der beiden rohrförmigen Formkörper werden mit einer aushärtbaren Kunststoffmasse gefüllt, wobei nach erfolgter Füllung die noch offenseitigen Enden der rohrförmigen Formkörper verschlossen werden. Härtet die Kunststoffmasse in den Innenräumen der rohrförmigen Formkörper aus, dann wird eine feste Verbindung und Fixierung der Stützstäbe und der Knochenschrauben erreicht. Damit wird eine Rahmenkonstruktion erhalten, vermittels dar eine orthopädische Fixierung und Knochenfragmente zusammengehalten werden und die darüber hinaus gleichzeitig ein Abstützsystem für das Pferdebein bildet, um den gebrochenen Pferdebeinabschnitt ruhigzustel-len und von einem Gewicht zu entlasten. Da Pferde bekanntlich stehen, muß für ein Zusammenheilen der gebrochenen Knochenfragmente eine Gewichtsentlastung des Pferdebeines erfolgen, und diese Gewichtsentlastung wird über die erhaltene Rahmenkonstruktion erreicht. Dieser bekannte Fixateur wird in der Weise verwendet, daß die Knochenschrauben oberhalb des gebrochenen Pferdebeines in den gesunden, ungebrochenen Knochen eingeschraubt werden, so daß dann der gebrochene Pferdebeinabschnitt unterhalb der Knochenschrau-benbefestigung völlig entlastet ist, da die Druckverteilung über die rohrförmigen Formkörper und die Stützstäbe in den Hufbereich des Pferdebeines erfolgt. Darüber hinaus besteht auch die Möglichkeit, Knochenschrauben oberhalb und unterhalb des gebrochenen Knochenabschnittes anzuordnen; jedoch auch bei dieser Ausführungsform erfolgt eine Abstützung und Gewichtsentlastung vermittels der Stützstäbe, die am Pferdehuf gehalten und an diesem befestigt sind. Ein derartiger Fixator ist beispielsweise nicht verwendbar, wo es sich um Fingerknochenbrüche handelt, und auch bei Knochenbrüchen des Beines läßt sich der bekannte Fixateur nicht verwenden, da bei einem Menschen keine Befestigungsmöglichkeiten des Fixateurs möglich ist, wie dies bei einem Pferd der Fall ist, bei dem der Fixateur am Huf des Pferde-beines befestigt wird.

Bei Fixateur externe aus flexiblen Bauteilen, die durch Aushärten zu einem starren System umgeandelt werden, wird ein mit flüssigem Kunststoff gefüllter Schlauch verwendet, der jedoch erst nach Fixation der Nägel am Knochen über die freien Enden desselben geschoben werden kann. In umgekehrter Folge würden nämlich die Nagelspitzen sowohl eine Infektion - ein derartiger Schlauch ist nicht sterilisierbar - als auch monomeren Kunststoff in den Organismus tragen. Durch diese vorgabe ist eine parallele Anordnung der Nägel erforderlich, was den Zuängen nahekommt, die das starre System dem Chirurgen auferlegt. Eine Fixation in mehr als einer Ebene, was aus mechanischen Erwägungen immer anzustreben ist, ist nicht mit einem Schlauch zu bewerkstelligen. In unterschiedlichem Ausmaß haben alle homogenen Kunststoffe nur eine geringe Bruchfestigkeit bei gleichzeitig niedriger Elastizität. Daraus resultiert die Notuendigkeit großer Querschnitte des tragenden Fixateurschlauches, ohne die Gewähr, daß der Fixateur aufgrund einer Spitzenbelastung nicht doch mal bricht. Hinzu kommt, daß eine Durchmischung der Kunststoffkomponenten in einem bereits gefüllten Schlauch mehrere Nachteile mit sich bringt. Die Gefahr einer unvollständigen Durchmischung mit der Folge, daß weiche und leichter abbiegbare Schlauchabschnitte nach Operationsende erhalten werden, ist relativ groß. Eine mangelnde Durchmischung erhöht darüber hinaus den Anteil verbleibender, unter Umständen toxischer Monomere im Fixateur. Eine möglichst gute Durchmischung ist nur in einem weichen, gut eindrückbaren Trägerschlauch möglich. Dieses hat zur Folge, daß ein möglichst konstanter Kunststoffquerschnitt nicht gewährleistet ist, insbesondere dann nicht, wenn der Fixateurschlauch in zahlreichen Windungen verlegt ist. Das Durchmischen der Komponenten, aus denen das aushärtbare Material besteht, bei bereits gefülltem Schlauch, führt praktisch immer zu einem Kunststoffaustritt aus den Nagelperforationsöffnungen und damit zu einem nicht kalkulierbaren Substanz- und Stabilitätsverlust. Beim Aushärten durch Zuführung von Wärme oder Strahlung entstehen noch folgende Probleme: Mit Strahlungen sind nur Flächen-Beschichtungen polymerisierbar. Ein vollständig gefüllter Schlauch ist mit Strahlung kaum polymerisierbar.

IR-Strahlung soll das nahe am Hautniveau - 2 bis 5 cm - liegende Schlauchsystem durchdringen, die Haut selbst jedoch nicht erwärmen. Der Schutz des Patienten vor UV-Strahlung stellt sich zwar einfacher dar als bei der Verwendung von IR-Strahlung, jedoch muß bei beiden Strahlungsarten eine umfassende Ausleuchtung des Schlauchsystems erreicht werden. Diese hat gleichzeitig mit einer Röntgendurchleuchtung und dem Repositionsmanöver des Chirurgen zu erfolgen, eine Gesamtanordnung, die unter Umständen technisch schwierig durchführbar ist. Im Falle eines fotosensiblen Kunststoffes, der durch eine lichtundurchlässige Folie geschützt ist, mag das Durchbohren des Schlauches mit Fixiernägeln bereits die Aushärtung auslösen. Eine abschließende Knochenreposition wird vom Chirurgen etwa 5 Min. gehalten. In dieser Zeit muß der Fixateur ausgehärtet sein. Längere Aushärtungszeiten erhöhen die Röntgenstrahlenbelastung und bergen die Gefahr eines eher schlechteren Repositionsergebnisses aufgrund der Ermüdung des Chirurgens.

Bei dem aus DE-A- 3141909 bekannten Fixateur ist eine einfache Handhabung ohne aufgezwungene Arbeitsebenen mit der Möglichkeit der Vornahme von Positionskorrekturen des Fixierschlauches vor Aushärten der Füllmasse, ohne daß es zu Verschiebungen ausgerichteter Knochenbruchstücken kommt, möglich, so daß auch komplizierte Knochenfrakturen, d.h. eine große Anzahl von Knochenbruchstücken, positionsgerecht gehalten werden können, bis es zur Aushärtung der Füllmasse gekommen ist, wobei eine universelle Anwendbarkeit gewährleistet und eine begrenzte Vorratshaltung mit kleinen Sortiment der Bauteile des Fixateur externe möglich ist.

Die Erfindung löst die Aufgabe, einen Fixateur externe gemäß der eingangs genannten Art zu schaffen, bei dem eine große Stabilität und Elastizität schon bei kleinen Querschnitten gegeben ist. Des weiteren ist es Aufgabe der Erfindung, für einen Fixateur externe ein schlauchförmiges Teil zu schaffen, das gleichzeitig als Gußform für den zu härtenden Kunststoff verwendbar ist und dessen Schlauchkörper einen innigen Verbund mit dem ausgehärteten Kunststoff eingeht.

Diese Aufgabe wird durch die in den Patentansprüchen 1 und 2 gekennzeichneten Merkmale gelöst.

Durch die Verwendung eines als Spiralschlauch ausgebildeten schlauchförmigen Teiles bei einem Fixateur externe aus einem Kunststoffschlauch mit einem eingezogenen Glasfasergewebe-Schlauch oder aus einem glasfaserverstärkten Kunststoff oder aus einem kunststoffeingebetteten Glasfasergewebe werden alle die bei den bekannten Fixateur externe gegebenen Nachteile behoben. Ein derart ausgebildeter schlauchförmiger Teil wird nach erfolgter Montage mit schnellhärtendem Kunstharz gefüllt, so daß aus einem flexiblen, leicht zu handhabenden Material ein starrer äußerer Festhalter erhalten wird, der zudem für Röntgenstrahlen durchlässig ist, der sehr kostengünstig und bei Verwendung verschiedener Schlauchstärken und Glasfaserfüllungen universell einsetzbar ist, so daß jede gewünschte Festigkeit erhalten wird. Die Verwendung des Fixateur externe als EinmalArtikel umgeht das Verschleißproblem. Durch den Einsatz eines Kunststoffglasfaserverbundes bei einem Fixateur externe wird erstmals ein Werkstoff eingesetzt, der es erstmals dem Operateur ermöglicht, ein System zur orthopädischen Fixierung und zum Zusammenhalten von Knochenfragmenten zur Verfügung zu haben, mit dem die an einen Fixateur externe gestellten Anforderungen erfüllt werden. Durch die Verwendung eines glasfasergefüllten Spiralschlauches wird ein Fixateurschlauch erhalten, der sich beim Aufbau des Fixateurs mühelos verformen und in verschiedene Ebenen abbiegen läßt, ohne daß es in den Knickstellen zu Querschnittsveränderunden kommt, was einerseits auf die gewisse Eigenstabilität des Fixateurschlauches und andererseits` darauf zurückzuführen ist, daß in den Kunststoffschlauch ein Glasfasergewebeschlauch eingezogen ist.

Gerade diese Formbeständigkeit beim Formen und Verlegen des Fixateurschlauches gewährleistet einen ungehinderten Durchfluß des eingegebenen, fließfähigen Kunststoffmaterials, das nach der Durchmischung der Komponenten außerhalb des Fixateurschlauches in diesen eingespritzt wird. In dem Fixateurschlauch kommt es dann zu einer "Durchmischung" von geueben Glasfasern mit dem Kunstharz und es entsteht ein Kunststoff-Glasfaser-Verbund. Die im Material des Fixateurschlauches verankerten Glasfasern bzw. der in den reinen Kunststoffschlauch eingezogene Glasfasergewebeschlauch erhöhen die Reißfestigkeit des schlauchförmigen Teiles, der als Spiral-schlauch ausgebildet: ist und sich aufgrund dieser Ausgestaltung mühelos abbiegen und somit verlegen läßt. Aufgrund der hohen Bruchfestigkeit. sind Schläuche mit kleinen Querschnitten auch verwendbar. Auch beim Einspritzen der vorzugsweise schnellhärtenden Kunstharzmasse ist ein Kunststoffaustritt aus den Nagelperforationsöffnungen in den Wänden des Fixateurschlauches nicht möglich, de die durch das Einführen der Fixiernägel oder -schrauben in die Wand des Fixateurschlauches gebildeten Öffnungen im Durchtrittsbereich des Fixiernagels sich selbsttätig wieder schließen, wobei dieses selbsttätige Verschließen unterstützt wird durch die in das Schlauchmaterial eingearbeiteten Glasfasern, die Glasfaserfüllung oder durch den eingezogenen Glasfasergewebe-Schlauch, die eine Art Verschlußsicherung darstellen, wobei es vorteilhaft ist, wenn schlauchförmige Teile verwendet werden, die aus einem in einen Kunststoff eingebetteten, sehr engmaschigen Glasfasergewebe oder aus einem sehr engmaschigen Glasfasergewebe-Schlauch bestehen. Dadurch ist auch sehr dünnwandiger Schlauch verwendbar.

Nach einer bevorzugten Ausführungsform Erfindung besteht der Fixateur externe aus einem einzigen schlauchförmigen Teil aus einem in einen Kunststoffschlauch eingezogenen Glasfasergewebe-Schlauch oder aus einem glasfaserverstärkten Kunststoff oder aus einem in einen Kunststoff eingebetteten Glasfasergewebe, wobei dieser schlauchförmige Teil in verschiedenen Ebenen und Richtungen über den Knochenfragmenten geführt und vermittels der Drähte, Nägel oder Schrauben mit den Knochenfragmenten verbunden ist. Die beiden freien Enden dieses schlauchförmigen Teiles sind über ein vorzugsweise Y- oder T-förmiges Verbindungsstück miteinander verbunden, das mit einer verschließbaren Einfüllöffnung für das aushärtbare Material versehen ist. Auf diese Weise ist es möglich, Bruchstücke gebrochener Knochen, z.B. der Fingerknochen, mit einem einzigen schlauchförmigen Teil unter Verwendung von Drähten, Nägeln oder Schrauben zu fixieren, ohne daß es hierzu eines aufwendigen Rahmengestells bedarf. Nach dem Einführen des schnellhärtenden Kunstharzes in den schlauchförmigen Teil und nach dem Aushärten des Kunstharzes wird ein festes, in sich starres System erhalten, so daß ein guter Heilungsprozeß erreicht wird.

Des weiteren besteht die Möglichkeit, mehrere schlauchförmige Teile zu einem Rahmen zusammenzufügen, wobei alle schlauchförmigen Teile über rohrförmige Verbindungsstücke derart miteinander verbunden sind, daß die Innenräume aller schlauchförmigen Teile miteinander verbunden sind, wobei das so ausgebildete Schlauchsystem mindestens eine verschließbare Einfüllöffnung für das aushärtbare Material aufweist. Darüber hinaus besteht auch die Möglichkeit, mehrere schlauchförmige Teile des Schlauchsystems gruppenweise zusammenzufassen, wobei das von jeder Gruppe gebildete Schlauchsystem mindestens eine verschließbare Einfüllöffnung für das aushärtbare Material aufweist.

Auf diese Weise ist es möglich, einen derartigen äußeren Festhalter den unterschiedlichen Erfordernissen optimal anzupassen, wobei verschiedene Arten von Verbindungsstücken zur Anwendung gelangen. Als zwei-dimensionale Verbindungsstücke finden dann Verwendung um 90° oder um 180° abgebogene Verbindungsstücke sowie ferner T-, Y- und Kreuz-Verbindungsstücke In frakturüberspannenden Schlauchabschnitten sollten vorzugsweise keine Verbindungsstücke eingearbeitet sein, da diese unter Umständen zu Schwachstellen der Stabilität werden könnten.

Um eine ausreichende Kraftübertragung vom Schlauchsystem zu den fixierenden Nägeln oder Schrauben zu gewährleisten, ist es bei größeren Nagel- oder Schraubenquerschnitten und gleichzeitig größerem Kräfteaufkommen, etwa bei Frakturen der unteren Extremität, nicht mehr ausreichend, die Nägel oder Schrauben einfach durch den Schlauch zu bohren, zumal ein Umbiegen und Versenken aufgrund des größeren Nagelquerschnittes nicht mehr möglich ist. Der Nagel oder die Schraube muß vielmehr durch ein Fixierungselement am Schlauchsystem verankert werden, wobei ein derartiges Fixierungselement folgende Aufgaben zu erfüllen hat:

- Es muß an jeder beliebigen Stelle des Schlauches anzubringen sein,
- es muß den Nagel oder die Schraube rotationsstabil halten können,
- es soll selbst im Glasfaserkunststoff des Schlauches verankert sein, um den Kraftschluß zu verteilen,
- es soll weitestgehend universell für verschiedene Nagel- oder Schraubenquerschnitte einsetzbar sein,
- das Element muß röntgentransparent sein.

Alle diese Voraussetzungen erfüllt ein Fixierungselement aus zwei schalenförmigen, den schlauchförmigen Teil umgebenden Teilen, die sich zu einen rohrförmigen Bauelement ergänzen, wobei jedes schalenförmige Teil mindestens an zwei sich gegenüberliegenden Seiten schalenförmig ausgebildete Abschnitte aufweist, die sich zu zwei miteinander fluchtenden Befestigungsstutzen ergänzen und zur Aufnahme einer Schraube oder eines Nagels dienen, wobei die schalenförmigen Abschnitte auf ihrer Außenwand ein Außengewinde zur Aufnahme einer einen Quetschring übergreifenden Überwurfmutter tragen. Neben Schrauben mit einem sich über die gesamte Länge des Schraubenschaftes erstreckenden Gewinde können auch Schrauben verwendet werden, die am eingebohrten Abschnitt ein Gewinde tragen, während der übrige Schaft glatt ausgebildet ist. Selbst dann, wenn das Gewinde durchgehend am Schraubenschaft t ausgebildet ist, ist die Handhabung dieser Schrauben in der Weise, als wenn kein Gewinde vorhanden sei. Das Ansetzen der klemmbackenartig wirkenden schalenförmigen Teile des Fixierungselementes erfolgt nach dem Einbohren der Nägel oder Schrauben.

Des weiteren besteht das Fixierungselement aus einem durch zwei sich gegenüberliegenden und miteinander fluchtenden Durchbrechungen in der Wand des schlauchförmigen Teiles hindurchgeführten, mit einem Außengewinde versehenen Rohr, das in seinem Innenraum einen Nagel oder eine Schraube aufnimmt, wobei das Gewinderohr beidseitig mit einem Abschnitt aus dem schlauchförmigen Teil herausgeführt ist und auf seinen Abschnitten im Bereich deren Enden je eine in das Außengewinde des Gewinderohres eingreifende Überwurfmutter trägt, die einen am Ende eines jeden Abschnittes des Gewinderohres vorgesehenen, an der Außenwandfläche des in dem Gewinderohr angeordneten Nagels oder Schraube zur Anlage beim Anziehen der Überwurfmutter bringbaren Quetschring übergreift, wobei das Gewinderohr auf seinem dem Knochenragment zugekehrten Abschnitt eine gewindetragende, auf dem Außengewinde aufsitzende Überwurfklammer mit in die Wand des schlauchförmigen Teiles eingreifenden Zapfen, Dorn od.dgl. und auf seinem anderen Abschnitt eine gewindefreie Überwurfklammer mit in die Wand des schlauchförmigen Teils eingreifendem Zapfen, Dorn od.dgl. trägt, wobei die gewindefreie Überwurfklammer auf dem Gewinderohr mittels einer Mutter gehalten ist.

Diese beiden unterschiedlich ausgebildeten Fixierungselemente passen sich den unterschiedlichen Nagel- oder Schraubenquerschnitten durch Verwendung entsprechender Quetschringe an und erfüllen somit die an das Fixierungselement gestellten Anforderungen.

Vorteilhafte Ausgestaltungen der Erfindung sind in den Unteransprüchen gekennzeichnet.

Im folgenden wird der Gegenstand der Erfindung in den Zeichnungen erläutert, in denen jedoch die Ausdildung als Spiralschlanch nicht in allen Figuren dargestellt ist. Es zeigt

Fig 1 in einer schaubildlichen Ansicht einen aus mehreren miteinander verbundenen schlauchförmigen Teilen bestehenden, an zwei Knochenbruchstücken angelegten Fixateur externe,

Fig. 2 in einer schaubildlichen Ansicht einen aus einem endseitig miteinander verbundenen schlauchförmigen Teil bestehenden und mittels Kirschner-Drähten an zwei Knochenbruchstükken befestigten Fixateur,

Fig. 3 teils in Ansicht, teils in einem senkrechten Schnitt einen an einem Abschnitt eines schlauchförmigen Teiles des Fixateur externe befestigten Kirschner-Drahtes,

Fig. 4 eine schaubildliche Ansicht eines Fixierungselementes,

Fig. 5 einen Teilquerschnitt durch das Fixierungselement in Fig 4,

Fig. 6 teils in Ansicht, teils in einem senkrechten Schnitt eine weitere Ausführungsform des Fixierungselementes,

Fig. 7 einen waagerechten Schnitt gemäß Linie VII-VII in Fig. 6

Fig. 8 einen Längsschnitt durch einen Perforator zum Einbringen des Gewinderohres in das Fixierungselement nach Fig. 6,

Fig. 9 eine schaubildliche Ansicht eines Spannelementes,

Fig. 10 eine Draufsicht auf das Spannelement in Richtung seiner Längsachse,

Fig. 11 einen Längsschnitt durch eine Hälfte des Spannelementes nach Fig. 9,

Fig. 12 in einem vergrößerten senkrechten Schnitt einen Abschnitt eines schlauchförmigen Teiles aus einem Schlauchkörper aus einem Kunststoff mit einem in den Schlauchkörper eingezogenen Glasfasergewebe-Schlauch,

Fig. 13 in einem vergrößerten, senkrechten Schnitt einen Abschnitt eines schlauchförmigen Teiles aus einem Schlauchkörper aus einem Kunststoff mit mehreren, schichtartig eingezogenen Glasfasergewebe-Schläuchen,

Fig. 14 in einem vergrößerten, senkrechten Schnitt einen Abschnitt eines schlauchförmigen Teiles aus einem Schlauchkörper aus einem Kunststoff mit einem in den Schlauchkörper eingezogenen Glasfasergewebe-Schlauch, wobei in dem Innenraum des schlauchförmigen Teiles ausgehärteter Kunststoff angeordnet ist,

Fig. 15 in einem vergrößerten, senkrechten Schnitt einen Abschnitt eines schlauchförmigen Teiles aus einem spiralförmigen, mit einem Glasfasergewebe gefüllten Schlauchkörper,

Fig. 16 einen senkrechten Schnitt gemäß Linie XVI-XVI in Fig. 15,

Fig. 17 in einem vergrößerten senkrechten Schnitt einen Abschnitt eines schlauchförmigen Teiles aus einem Schlauchkörper aus Kunststoff mit in diesem eingebetteten Glasfasern und

Fig. 18 in einem vergrößerten, senkrechten Schnitt einen Abschnitt eines schlauchförmigen Teiles aus einem Schlauchkörper aus einem Kunststoff mit einem in diesem eingearbeiteten Glasfasergewebe.

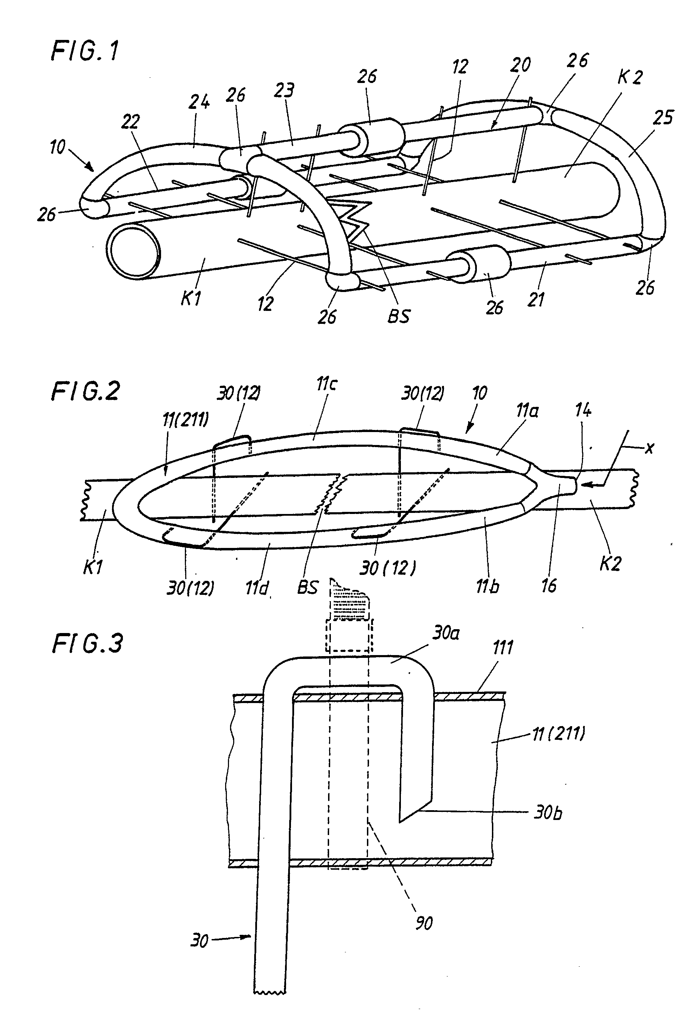

Der in Fig 1 und 2 dargestellte Fixateur externe ist mit 10 bezeichnet. Dieser Fixateur externe 10 besteht aus einem schlauchförmigen Teil 11 bzw 211 in Form eines Schlauchsystems 20 aus mehreren einzelnen, miteinander verbundenen Schlauchabschnitten, dessen beiden Enden miteinander verbunden sind (Fig. 2). Dieser schlauchförmige Teil 11 bzw. 211 bildet den Fixateurschlauch; seine Wand ist mit 111 bzw. 211a bezeichnet. Die Befestigung des schlauchförmigen Teiles 11 bzw. 211 an zwei mit K1 und K2 bezeichneten Knochenbruchstücken erfolgt mittels Drähten, Nägeln oder Schrauben 12.

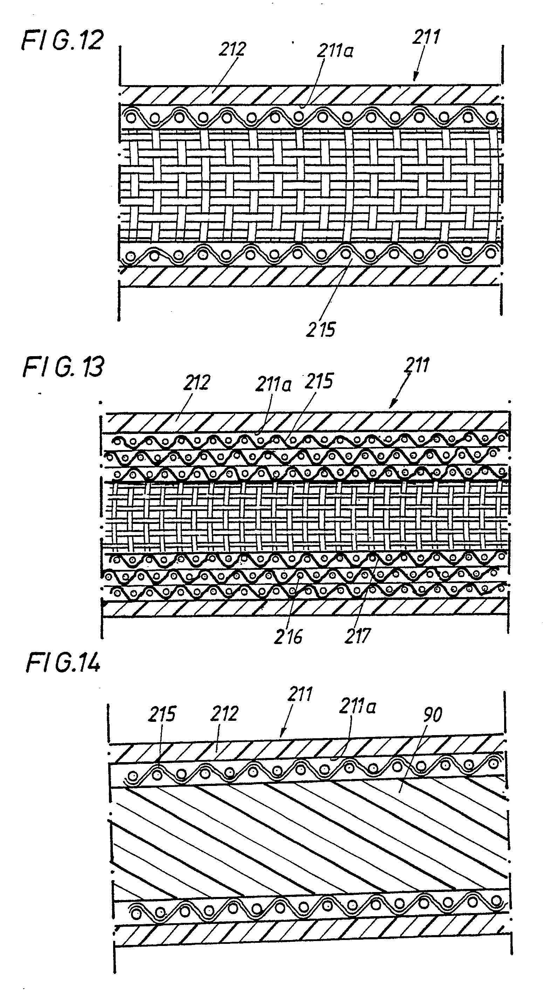

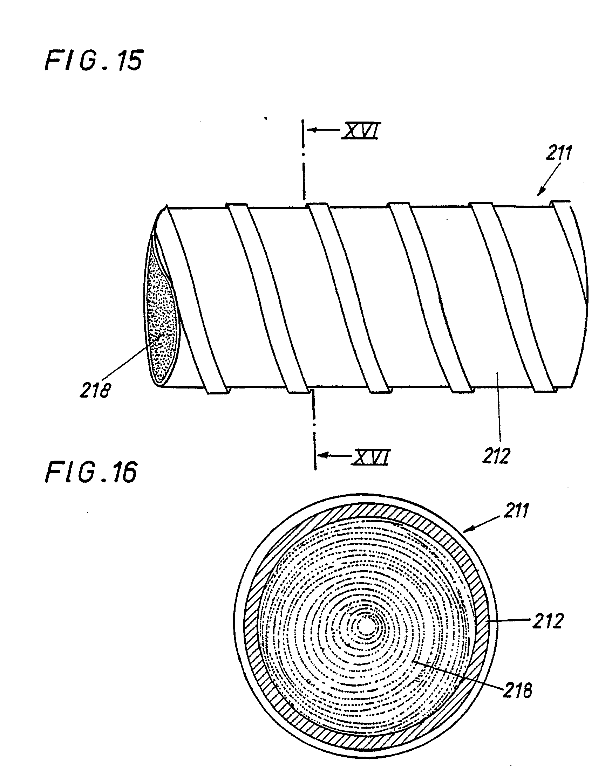

Der schlauchförmige Teil 211 besteht aus einem Schlauchkörper 212 aus einem flexiblen, eine gewisse Elastizität aufweisenden Kunststoff. Dieser Schlauchkörper 212 ist mit Glasfasern oder anderen mineralischen Fasern gefüllt. Diese Glasfaserfüllung erfolgt in Form eines in den Innenraum des Schlauchkörpers 212 eingezogenen Schlauches bzw. Strumpfes 215 aus einem Glasfasergewebe (Fig. 12) Entsprechend Fig. 13 können auch mehrere einzelne Glasfasergewebe-Schläuche 215,216,217 in den Innenraum des Schlauchkörpers 212 eingezogen sein, so daß sich im Querschnitt gesehen mehrere Schichten aus Glasfasergeweben ergeben. Nach einer weiteren Ausführungsform nach Fig. 15 und 16 sind in den Innenraum des Schlauchkörpers 212 auch so viele Glasfasergewebe-Schläuche eingezogen, daß der Innenraum des Schlauchkörpers 212 vollständig mit Glasfasern gefüllt ist. Dabei besteht auch die Möglichkeit, anstelle von Glasfasergewebeschläuchen eine Glasfasergewebematte zu verwenden, die gefaltet oder zusammengerollt in den Innenraum des Schlauchkörpers 212 eingezogen wird. Bei der Verwendung mehrerer Glasfasergewebeschläuche können alle Schläuche die gleiche Stärke aufweisen; jedoch auch unterschiedliche Stärken können zur Anwendung gelangen.

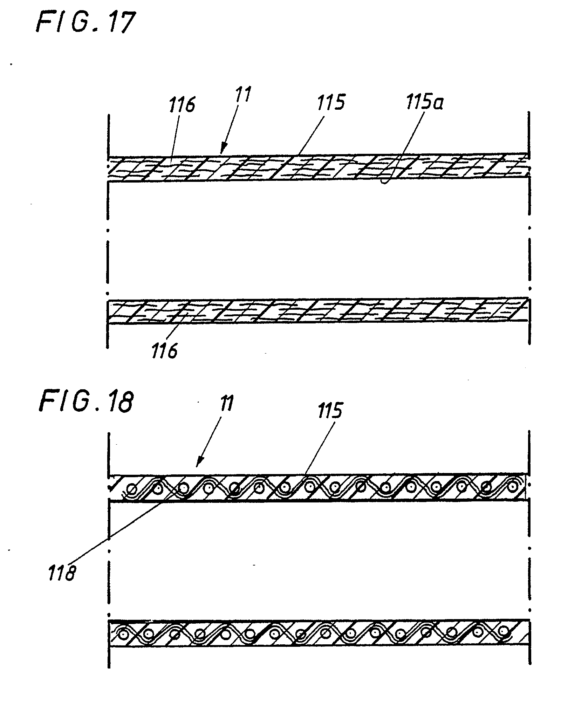

Nach einer weiteren Ausführungsform gemäß Fig. 17 und 18 besteht der schlauchförmige Teil 11 aus einem glasfaserverstärkten Kunststoff oder aus einem in einen Kunststoff eingebetteten Glasfasergewebe. Der schlauchförmige Teil 11 umfaßt hiernach einen Schlauchkörper 115 aus einem flexiblen, eine gewisse Elastizität aufweisenden Kunststoff, in den zur Verstärkung Glasfasern 116 eingearbeitet sind, wobei es vorteilhaft ist, wenn aus der Innenwandfläche 115a Glasfasern heraustreten oder zumindest in der Oberfläche der Innenwand 115a liegen, um als eine Art Haftvermittler mit dem im Innenraum des Schlauchkörpers 115 ausgehärteten Kunststoff einen innigen, festen Verbund eingehen zu können.

Es besteht auch die Möglichkeit, anstelle von in den Kunststoff des Schlauchkörpers 115 eingearbeitete Glasfasern ein Glasfasergewebe 118 in den Kunststoff einzuarbeiten, wobei auch bei dieser Ausführungsform das Glasfasergewebe oder eine eingearbeitete Glasfasermatte in der Oberfläche der Innenwand des Schlauchkörpers 115 liegen sollte (Fig. 18). Neben Glasfasern können auch in das Kunststoffmaterial des schlauchförmigen Teils 11 Mineralfasern oder Fäden eingearbeitet sein, worunter alle Fasern verstanden werden, die aus anorganischen Rohstoffen hergestellt werden. Besonders vorteilhaft ist jedoch hier der Einsatz von Glasfasern und -fäden, die auch in Form von Geweben oder Matten in das Kunststoffmaterial des schlauchförmigen Teiles 11 eingearbeitet sein können oder in Form eines in den Kunststoffschlauchkörper eingezogenen Glasfasergewebe-Schlauches vorliegen. Besonders vorteilhaft ist die Verwendung glasfaserverstärkter Thermoplaste, die mit Glas-Kurzfasern von weniger als 1 mm oder mit Glasfasern bis zu etwa 3 mm Länge ausgerüstet sind, wobei jedoch auch Glasfasern größerer Länge zum Einsatz gelangen können. Als glasfaserverstärkte Thermoplaste können eingesetzt werden ABS, Polyamide, PC,PE.POM,PP,PS,PVC,SAN,PETP,PBTP, wobei im wesentlichen diejenigen Kunststoffe bzw. Thermoplaste zum Einsatz gelangen, die sich zur Herstellung der schlauchförmigen Teile 11 für den Fixateur externe 10 eignen und auch in Verbindung mit der Hautnähe eine gute Hautverträglichkeit besitzen. Als Glasfasergewebe können Flächengebilde aus Glasseidenrovings, Glasfäden, Glasgespinsten oder Glasfaservliesen eingesetzt werden. Dabei können die Glasfasergewebe die verschiedensten Bindungstypen aufweisen. So können z.B. Zweischaftbindungen, Köperbindungen und Atlasbindungen Verwendung finden, jedoch auch alle anderen Arten von geeigneten Bindungen können für die Herstellung des Glasfasergewebes herangezogen werden.

Zur Herstellung des Fixateur externe 10 wird der schlauchförmige Teil 11 bzw. der schlauchförmige Teil 211, der mit Glasfasern gefüllt ist, nach dem Anlegen und Fixieren der Knochenbruchstücke mit einem aushärtbaren Material gefüllt. Insbesondere kommen solche Kunststoffe zur Anwendung, die schnellhärtend sind, da nur durch die Verwendung schnellhärtender Kunststoffe gewährleistet wird, daß die fixierten Knochenbruchstücke nach dem Aushärten ihre vorgegebene Position beibehalten Außerdem wird dem Operateur durch die kurze Aushärtezeit die Möglichkeit gegeben, die ausgerichteten Knochenbruchstücke in der ausgerichteten Position zu halten, ohne daß es hierbei seitens des Operaters zu Ermüdungserscheinungen kommt, die zu einer nicht mehr positionsgerechten Anordnung der Knochenbruchstücke führen können. Vorzugsweise wird schnell aushärtbares Material eingesetzt, wobei jedoch auch zur Verkürzung der A ushärtezeit Licht eingesetzt werden kann, wenn ein entsprechendes aushärtbares Material eingesetzt wird. Der schnellhärtende Kunststoff wird außerhalb des Fixateurschlauches durch Vermischen der beiden Komponenten (Härter und Kunststoffmasse) vorbereitet und dann in den Fixateurschlauch injiziert. Vorteilhaft ist es, wenn der Schlauchkörper 212 aus einem transparenten bzw. glasklaren Kunststoff besteht und wenn die einzugebende Kunststoffmasse eingefärbt ist. Beim Eingeben dieser eingefärbten Kunststoffmasse kann dann die Verteilung der Kunststoffmasse im Innenraum des Schlauchkörpers beobachtet und überwacht werden, ob alle Abschnitte des Schlauchkörpers 212 mit Kunststoffmasse ausgefüllt sind.

Anstelle der in den nachstehenden Ausführungsbeispielen verwendeten schlauchförmigen Teilen 11 in den in Fig. 17 und 18 gezeigten Ausführungsformen können auch schlauchförmige Teile 211 eingesetzt werden, die den in Fig. 12, 13, 15 und 16 gezeigten und vorangehend beschriebenen Aufbau aufweisen.

Bei dem in Fig 2 gezeigten Ausführungsbeispiel besteht der Fixateur externe 10 aus einem schlauchförmigen Teil 11, dessen beiden Enden 11a, 11b in einem Verbindungsstück 16 zusammengeführt sind, das mit einer verschließbaren Einfüllöffnung 14 versehen ist. Die Einführung des gieß- bzw. fließfähigen Kunststoffmaterials in den Innenraum des schlauchförmigen Teiles 11 erfolgt in Pfeilrichtung X. Der schlauchförmige Teil 11 ist mittels Kirschner-Drähten 30 mit den beiden Knochenbruchstücken K1, K2 verbunden, d.h. der schlauchförmige Teil 11 ist mittels dieser Kirschner-Drähte an den beiden Knochenbruchstücken K1, K2 befestigt, und zwar derart, daß der schlauchförmige Teil 11 in einem geringen Abstand oberhalb der beiden Knochenbruchstücke zu liegen kommt. Sind mehrere Knochenbruchstücke zu fixieren, dann ist dieser schlauchförmige Teil 11 in verschiedenen Ebenen und Richtungen über diese Knochenbruchstücke geführt, wobei dann die Befestigung des schlauchförmigen Teils 11 mit den einzelnen Knochenbruchstücken ebenfalls über Kirschner-Drähte 30 oder andere geeignete Drähte oder Stifte erfolgt, wobei dann die Kirschner-Drähte einerseits in die einzelnen Knochenbruchstücke eingreifen und mit ihren anderen Enden mit demjenigen Abschnitt des schlauchförmigen Teiles 11 verbunden sind, der dem zu fixierenden Knochenbruchstück zugewandt ist. Da der schlauchförmige Teil 11 bei der in Fig. 2 gezeigten Ausführungsform nur oberhalb und auf einer Seite der Knochenbruchstücke angeordnet ist, sind die Kirschner-Drähte 30 nur einseitig in den einzelnen Knochenbruchstücken befestigt. Die durch die Knochenbruchstücke hindurchgeführten Abschnitte der Kirschner-Drähte sind mit ihren freien Enden dann nicht mit einem entsprechenden schlauchförmigen Teil 11 verbunden, jedoch besteht auch hier die Möglichkeit, zwei schlauchförmige Teile 11 zu verwenden, von denen der eine schlauchförmige Teil 11 oberhalb der zu fixierenden Knochenbruchstücke und der andere schlauchförmige Teil 11 unterhalb der zu fixierenden Knochenbruchstücke zu liegen kommt, so daß dann die beiden schlauchförmigen Teile 11 bei gleichzeitigem Hindurchführen der Kirschner-Drähte 30 durch die Knochenbruchstücke miteinander verbunden sind. Die Anordnung des schlauchförmigen Teiles 11 oberhalb der zu fixierenden Knochenbruchstücke K1, K2 kann derart sein, daß die Kirschner-Drähte 30 alle in der gleichen Richtung in die Knochenbruchstücke eingeführt sind. Wie Fig. 2 zeigt, kann der schlauchförmige Teil 11 jedoch so angeordnet sein, daß z.B. zwei Kirschner-Drähte 30 in senkrechter Richtung durch den Abschnitt 11c des schlauchförmigen Teiles 11 hindurchgeführt sind, während der andere Abschnitt 11d des schlauchförmigen Teiles 11 derart zum Abschnitt 11c abgebogen ist, daß die durch diesen Schlauchabschnitt 11d hindurchgeführten Kirschner-Drähte 30 quer zur Längsrichtung der durch den Abschnitt 11c des schlauchförmigen Teiles 11 hindurchgeführten Kirschner-Drähte zu liegen kommen. Für eine derartige Anordnung und Zuordnung der beiden Abschnitte 11c, 11 d des schlauchförmigen Teiles 11 kann dann das verwendete Verbindungsstück 16 eine entsprechende Formgebung aufweisen. Der schlauchförmige Teil 11 besteht auch bei dieser Ausführungsform aus einem glasfasergefüllten Kunststoff oder aus einem in einen Kunststoff eingebetteten Glasfasergewebe, wobei der schlauchförmige Teil 11 auch als Spiralschlauch ausgebildet sein kann. Die Befestigung des schlauchförmigen Teiles 11 an den Knochenbruchstücken K1, K2 kann mit Kirschner-Drähten 30, jedoch auch mit andersartig ausgebildeten Drähten, Nägeln oder Schrauben, erfolgen.

Um eine rotationsstabile Verankerung der Kirschner-Drähte 30 an dem schlauchförmigen Teil 11 zu erreichen, ist das freie Ende eines jeden Kirschner-Drahtes 30 um etwa 180° U-förmig abgebogen. Dieses abgebogene Drahtende ist mit 30a bezeichnet (Fig. 3). Das freie Ende 30b des abgebogenen Drahtabschnittes 30a kann angeschrägt oder spitz verlaufend ausgebildet sein, so daß ein müheloses Einstechen und Einführen in die Wand 111 des schlauchförmigen Teiles 11 möglich ist.

Bei der in Fig. 1 dargestellten Ausführungsform eines Fixateur externe 10 wird aus einer Anzahl einzelner miteinander verbundener schlauchförmiger Teile 11 ein Rahmen 20 geschaffen, in den die beiden Knochenbruchstücke K1, K2 vermittels Nägel, Schrauben, Stifte oder Drähte 12 eingespannt und fixiert sind. Dieser Rahmen 20 wird aus drei Längsholmen 21, 22, 23 gebildet, von denen z.B. jeder Längsholm aus zwei miteinander verbundenen Abschnitten besteht. Diese drei Längsholme 21, 22, 23 sind über halbkreisförmige Bögen 24, 25 im Bereich ihrer freien Enden verbunden, wobei auch diese halbkreisförmigen Bögen 24, 25 aus je zwei kreisbogenförmig ausgebildeten und miteinander verbundenen Abschnitten bestehen. Die Längsholme 21, 22, 23, die halbkreisförmigen Bögen 24, 25 und auch die einzelnen Abschnitte, aus denen die Längsholme und die halbkreisförmigen Bögen gebildet sind, sind über Verbindungsstücke 26 miteinander verbunden, wobei diese Verbindungsstücke 26 ebenso auch wie das Verbindungsstück 16 bei dem in Fig. 2 gezeigten Fixateur externe rohrförmig ausgebildet sind. Diese Verbindungsstücke 26 sind als T- oder Winkel-Stück ausgebildet. Werden zwei Holmabschnitte miteinander verbunden, so ist das Verbindungsstück lediglich rohrförmig ausgebildet. Wesentlich ist, daß alle schlauchförmigen Teile, die zu dem Rahmen 20 vermittels der Verbindungsstücke 26 zusammengefügt sind, so über diese Verbindungsstücke 26 miteinander verbunden sind, daß ein frei zugänglicher Innenraum geschaffen wird. Zum Einführen des aushärtbaren Materials in den Innenraum dieses Rahmens 20 aus den verschiedenen schlauchförmigen Teilen 11 ist eine in der Zeichnung nicht dargestellte verschließbare Einfüllöffnung vorgesehen. Es besteht jedoch auch die Möglichkeit, mehrere Einfüllöffnungen vorzusehen, die dann vorzugsweise über entsprechende Verbindungsleitungen mit einer einzigen Füllvorrichtung verbunden sein sollten, damit ein gleichmäßiges Einführen des aushärtbaren Materials gewährleistet ist, um zu erreichen, daß insbesondere wenn es sich um schnellhärtende Kunststoffe handelt, dann fast zu gleicher Zeit in allen Innenraumabschnitten des Rahmens 20 Kunststoff eingeführt und enthalten ist.

Bei dem in Fig. 1 gezeigten Ausführungsbeispiel ist der Rahmen 20 aus den schlauchförmigen Teilen 11 derart ausgebildet, daß Nägel, Schrauben oder Drähte 12 in senkrechter Richtung verlaufend angeordnet werden können, wobei diese in senkrechter Richtung verlaufenden und in dem Längsholm 23 gehaltenen Stifte 12 nur in den Knochenbruchstücken eingelassen sind, während die durch die in einer Ebene liegenden Längsholme 21, 22 hindurchgeführten Stifte oder Nägel 12 durch die Knochenbruchstücke K1, K2 hindurchgeführt sind. Es besteht jedoch auch die Möglichkeit, den Rahmen 20 so auszubilden, daß die beiden zu fixierenden Knochenbruchstücke K1, K2 allseitig von dem Rahmen 20 dann umgeben sind, so daß die durch die Knochenbruchstücke hindurchgeführten Nägel, Stifte oder Schrauben 12 jeweils beidendseitig in den den Rahmen 20 bildenden Längsholmen angeordnet sind.

Es besteht darüber hinaus auch die Möglichkeit, mehrere schlauchförmige Teile 11 des Rahmens 20, d.h. des so gebildeten Schlauchsystems gruppenweise zusammenzufassen, wobei das von jeder Gruppe gebildete Schlauchsystem mindestens eine verschließbare Einfüllöffnung für das aushärtbare Material aufweist. Das Füllen mit aushärtbarem Material kann in allen ausgebildeten einzelnen Schlauchsystemen gleichzeitig oder auch in kurzer Zeitfolge hintereinander vorgenommen werden.

Der zwischen den beiden Knochenbruchstücken K1, K2 ausgebildete Bruchspalt ist in den Fig. 1 und 2 bei BS angedeutet.

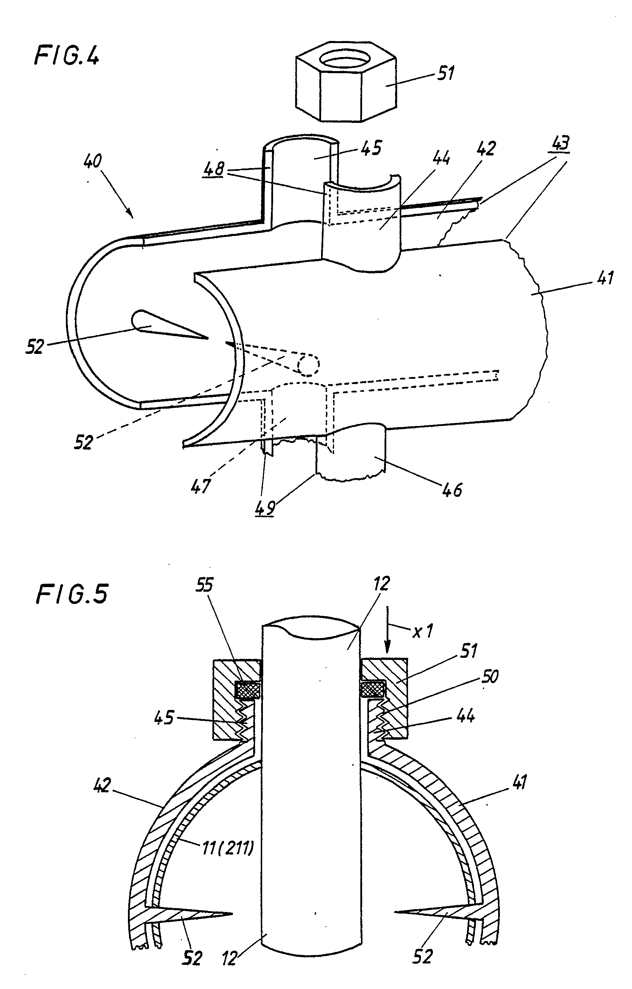

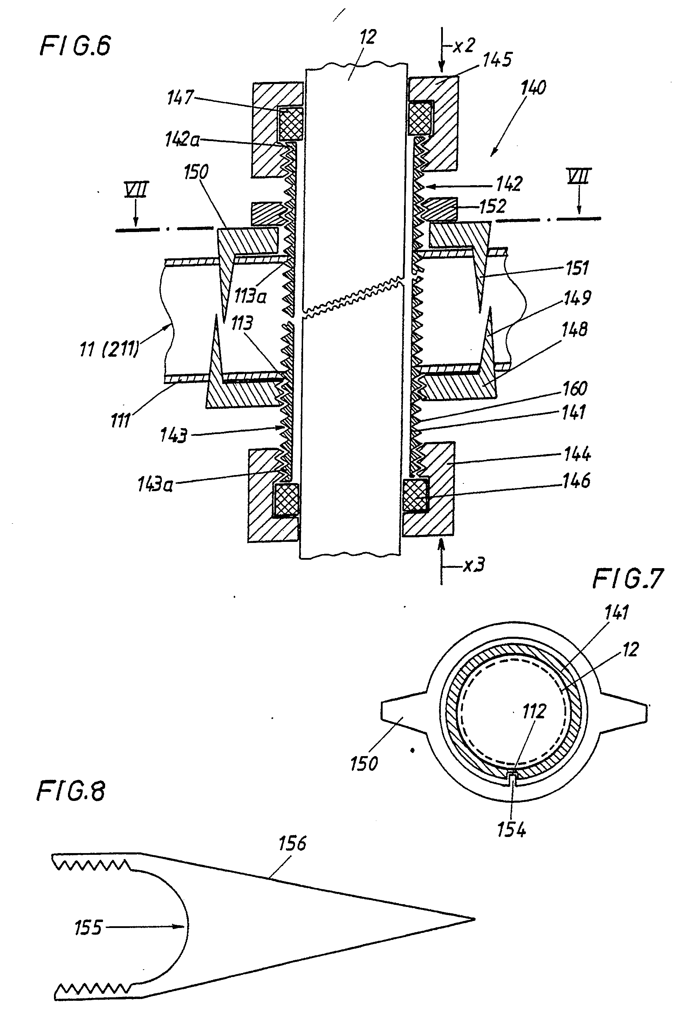

Zur Verankerung der Nägel oder Schrauben an dem aus den schlauchförmigen Teilen 11 bestehenden Schlauchsystem ist ein Fixierungselement 40 vorgesehen, das die in Fig. 4 und 5 dargestellte Ausbildung aufweist. Dieses Fixierungselement 40 besteht aus zwei etwa halbkreisbogenförmig ausgebildeten schalenförmigen Teilen 41, 42, die sich zu einem rohrförmigen Bauelement 43 ergänzen, wobei dieses rohrförmige Bauelement 43 einen Durchmesser aufweist, der in etwa dem Außendurchmesser des schlauchförmigen Teiles 11 entspricht. Neben schlauchförmigen Teilen 11 mit einem kreisförmigen Querschnitt können auch schlauchförmige Teile mit einem ovalen Querschnitt Verwendung finden. Die beiden, das rohrförmige Bauelement 43 bildenden schalenförmigen Teile 41, 42 des Fixierungselementes 40 sind der Außenformgebung des verwendeten schlauchförmigen Teiles 11 entsprechend ausgebildet.

Jedes schalenförmige Teil 41 bzw. 42 des Fixierungselementes 40 weist an zwei sich gegenüberliegenden Seiten angeformte und schalenförmig ausgebildete Abschnitte 44, 45 bzw. 46, 47 auf, die sich zu zwei miteinander fluchtenden Befestigungsstutzen 48, 49 ergänzen und die zur Aufnahme einer Schraube oder eines Nagels 12 dienen. Die schalenförmigen Abschnitte 44, 45 bzw. 46, 47 tragen auf ihrer Außenwand ein Außengewinde 50, auf das eine Überwurfmutter 51 aufgeschraubt ist (Fig. 4 und 5). Die Halterung und Befestigung eines Nagels, Stiftes oder Schraube 12 erfolgt unter Verwendung eines Quetschringes 55, der von der auf das Außengewinde der Befestigungsstutzen 48 bzw. 49 aufgeschraubten Überwurfmutter 51 umgriffen wird. Die Anordnung des Quetschringes 55 ist dabei so getroffen, daß der Quetschring 55 auf dem freien Ende des einen kreisförmigen Querschnitt aufweisenden Befestigungsstutzens 48 bzw. 49 aufliegt, so daß die aufgeschraubte Überwurfmutter 51 den Quetschring 55 übergreift. Beim Anziehen der Überwurfmutter 51 wird der Quetschring in Pfeilrichtung X1 zusammengedrückt, mit der Folge, daß er sich gleichzeitig an die Außenwandfläche des Nagels 12 anlegt, und zwar unter Ausbildung eines Preßdrucks, so daß der Nagel 12 fest in dem Fixierungselement 50 gehalten ist. Die Wand 111 des in dem Fixierungselementes 40 angeordneten schlauchförmigen Teiles 11 weist in Verlängerung der beiden Befestigungsstutzen 48, 49 entsprechende Durchbrechungen auf, die vor dem Einführen des Nagels 12 ausgebildet sein können Da jedoch der schlauchförmige Teil aus einem glasfaserverstärkten Kunststoff oder aus einem in einen Kunststoff eingebetteten Glasfasergewebe mit einem gewissen Rückstellvermögen besteht, dehnt sich das Material des schlauchförmigen Teiles 11 beim Eindrücken des Nagels 12 derart, daß der Nagel 12, mühelos durch die Wand 111 des schlauchförmigen Teiles 11 hindurchgedrückt werden kann, so daß auf diese Weise auch Nägel mit größeren Durchmessern mühelos befestigt werden können. Die Befestigungsstutzen 48, 49 des Fixierungselementes 40 sind in bezug auf ihre Durchmesser den Durchmessern angepaßt, die die verwendeten Nägel oder Schrauben aufweisen. Dadurch, daß die verwendeten Quetschringe 55 an den Befestigungsstutzen 48, 49 eine ausreichende Preßkraft beim Anziehen der Überwurfmuttern 51 entwickeln, besteht auch die Möglichkeit, Nägel und Schrauben in den Befestigungsstutzen 48, 49 sicher zu halten, wenn die verwendeten Nägel oder Schrauben 12 gegenüber dem Innendurchmesser der Befestigungsstutzen 48, 49 einen kleineren Durchmesser aufweisen. Die Befestigungsstutzen 48, 49 sind gleich ausgebildet und weisen auch gleiche Querschnittsflächen und Durchmesser auf.

Zur Fixierung des in dem Fixierungselement 40 angeordneten schlauchförmigen Teiles 11 weisen die schalenförmigen Teile 41, 42, die das rohrförmige Bauelement 43 bilden, an ihrer Innenwandfläche Fixierungszapfen, -dorne od.dgl 52 auf, die beim Anlegen des Fixierungselementes 40 in die Wand 111 des schlauchförmigen Teiles 11 eingedrückt werden. Die Anzahl dieser Fixierungszapfen, -dorne od dgl. 52 kann beliebig sein. Es ist jedoch ausreichend, wenn im Bereich der beiden Enden des Fixierungselementes 40 je zwei sich gegenüberliegende Fixierungszapfen 52 vorgesehen sind (Fig. 4 und 5).

Wird das Fixierungselement 40 in Verbindung mit Stiften, Nägeln oder Drähten 12 verwendet, dann können die Innenwandflächen der beiden Befestigungsstutzen 48, 49 glatt ausgebildet sein. Werden dagegen Schrauben oder Stifte bzw. Nägel mit endseitig ausgebildeten Gewinden verwendet, dann ist es vorteilhaft, wenn die Befestigungsstutzen 48, 49 an ihren Innenwandflächen ein Innengewinde tragen, so daß eine zusätzliche Befestigung der Schrauben 12 möglich ist. Da jedoch die Befestigung und Halterung vermittels der Quetschringe 55 erfolgt, ist es jedoch nicht erforderlich, daß die Befestigungsstutzen 48, 49 an ihrer Innenwandfläche mit einem Gewinde versehen sind. In gleicher Weise können Nägel, Schrauben oder Stifte 12 mit dem Fixierungselement 40 gehalten werden.

Bei einer weiteren Ausführungsform eines Fixierungselementes 140 gemäß Fig. 6 besteht dieses Fixierungselement aus einem mit einem Außengewinde 160 versehenen Rohr 141, das in seinem Innenraum einen Nagel oder eine Schraube 12 zur Fixierung mit den Knochenbruchstücken aufnimmt. Diese Gewinderohr 141 wird durch die Wand 111 eines schlauchförmigen Teiles 11 hindurchgeführt. Hierzu ist es vorteilhaft, wenn in der Wand 111 des schlauchförmigen Teiles 11 zwei miteinander fluchtende Durchbrechungen 113,113a ausgebildet sind.

Das Gewinderohr 141 des Fixierungselementes 140 ist dann in dem schlauchförmigen Teil 11 derart angeordnet, daß das Gewinderohr 141 beidseitig mit einem Abschnitt 142 bzw. 143 aus diesem schlauchförmigen Teil 11 herausragt. Das auf der Außenwandfläche des Gewinderohres 141 ausgebildete Außengewinde 160 kann sich dabei über die gesamte Länge des Rohres 141 erstrecken. Es besteht jedoch auch die Möglichkeit, nur im Bereich der beiden Enden 142a, 143a der beiden Gewinderohrabschnitte 142, 143 Außengewinde vorzusehen

Das Gewinderohr 141 trägt auf seinen Abschnitten 142, 143 im Bereich deren Enden 142a, 143a bzw. an seinen Enden je eine in das Außengewinde 160 des Gewinderohres 141 eingreifende Überwurfmutter 144 bzw. 145. Jede Überwurfmutter 144 bzw. 145 übergreift einen am Ende eines jeden Abschnittes 142, 143 des Gewinderohres 141 vorgesehenen, an der Außenwandfläche des in dem Gewinderohr 141 angeordneten Nagels oder Schraube 12 zur Anlage beim Anziehen der Überwurfmutter 144 bzw. 145 bringbaren Quetschring 146 bzw. 147 (Fig. 6). Auf dem Gewinderohr 141 sind dann endseitig die Quetschringe 146, 147 mit den diese übergreifenden Überwurfmuttern 144, 145 angeordnet Bei einem Anziehen der Überwurfmuttern 144, 145 in Richtung der Pfeile X2, X3 werden die Quetschringe 146, 147 derart zusammengepreßt, daß sie zur Anlage an den Nagel oder der Schraube 12 gebracht werden. Die Quetschringe 55 und 146, 147 bestehen aus geeignetem, verformbaren Material, wie Gummi oder Kunststoffen, das nach Möglichkeit keine Eindrückungserscheinungen aufweist.

Des weiteren trägt das Gewinderohr 141 auf seinem Abschnitt 143 eine gewindetragende Überwurfklammer 148 und auf seinem Abschnitt 142 eine gewindefreie Überwurfklammer 150. Die gewindetragende Überwurfklammer 148 ist ringförmig ausgebildet und mit einem Innengewinde versehen, so daß die Überwurfklamme r 148 auf das Außengewinde 160 des Gewinderohres 141 aufschraubbar ist. Diese Überwurfklammer 148 weist mindestens zwei miteinander fluchtende Zapfen, Dorne od.dgl. 149 auf, die in Richtung zu dem schlauchförmigen Teil 11 zeigen und in die Wand 111 des schlauchförmigen Teiles 11 eingreifen. Damit die Zapfen, Dorne od.dgl. 149 der Überwurfklammer 148 in die Wand 111 des schlauchförmigen Teiles 11 eingreifen, wird nicht die Überwurfklammer 148 auf dem Außengewinde 160 des Gewinderohres 141 aufgeschraubt, sondern durch Drehen des Gewinderohres 141 um seine Längsachse wird die Überwurfklammer 148 so weit an die Wand 111 des schlauchförmigen Teiles 11 herangeführt, daß die Zapfen, Dorne od.dgl. 149 an der Überwurfklammer 148 in die Wand 111 des schlauchförmigen Teiles 11 eingreifen.

Aus diesem Grunde weist die Überwurfklammer 150 auf dem Abschnitt 142 des Gewinderohres 141 kein Gewinde auf, sondern diese Überwurfklammer 150, die ebenfalls ringförmig ausgebildet ist, wird vermittels einer Mutter 152 an die Wand 111 des schlauchförmigen Teiles 11 heranbewegt. Die Mutter 152 ist hierzu auf das Außengewinde 160 des Gewinderohres 141 aufgeschraubt. Auch die Überwurfklammer 150 weist zwei zueinander fluchtende Zapfen, Dorne od.dgl. 151 auf, die die in Fig. 6 gezeigte Stellung einnehmen und die, wenn die Überwurfklammer 150 an die Wand 111 des schlauchförmigen Teiles 11 herangeführt ist, in die Wand 111 eingreifen. Die beiden Überwurfklammern 148, 150 sind hiernach derart auf dem Gewinderohr 141 gehalten, daß sich ihre Zapfen, Dorne od.dgl. 149, 151 gegenüberliegend sind.

Dieses Fixierungselement 140 wird in der Weise angewandt, daß das Gewinderohr 141 durch entsprechend ausgebildete Durchbrechungen in der Wand des schlauchförmigen Teiles 11 hindurchgeführt wird. Hieraufhin werden dann die Überwurfklammern 148, 150 aufgesetzt und durch Drehen des Gewinderohres 141 die gewindetragende Überwurfklammer 148 an die Wand des schlauchförmigen Teiles 11 heranbewegt, so daß seine Zapfen, Dorne od.dgl. 149 in die Wand des schlauchförmigen Teiles 11 eingreifen bzw. durch die Wand 111 hindurchgeführt werden, was aufgrund des verwendeten Materials für den schlauchförmigen Teil 11 möglich ist, ohne daß nachher an diesen Einstichstellen beim Einführen des aushärtbaren Materials in diesen Bereichen Material ausfließt oder aus diesen Einstichstellen herausgedrückt wird.

In gleicher Weise wird die gewindefreie Überwurfklammer 150 auf das Gewinderohr 141 aufgesetzt und vermittels der aufgeschraubten Mutter 152 an die Wand 111 des schlauchförmigen Teiles 11 bewegt, so daß die Zapfen, Dorne od.dgl. 151 der Überwurfklammer 150 in gleicher Weise wie die Zapfen, Dorne od.dgl. 149 in die Wand 111 des schlauchförmigen Teiles 11 eingreifen (Fig. 6).

Die Überwurfklammer 150 ist ferner mit einer Rotationsbremse versehen, und zwar in Form eines an der Innenwandfläche der Überwurfklammer 150 ausgebildeten oder angeformten Nockens 154, der in eine in der Außenwandfläche des schlauchförmigen Teiles 11 bei 112 ausgebildeten Ausnehmung eingreift (Fig. 7). Die Rotationsbremse liegt dabei zwischen der Überwurfklammer 150 und dem Gewinderohr 141, damit zwischen dem Nagel und dem schlauchförmigen Teil 11 nach Füllung und Aushärtung der Kunststoffmasse eine Rotationsstabilität erzeugt wird.

Zum Einbringen des Gewinderohres 141 ist ein Perforator 155 vorgesehen, dessen Spitze bei 156 angedeutet ist. Dieser Perforator wird zum Einbringen des Gewinderohres 141 aufgeschraubt.

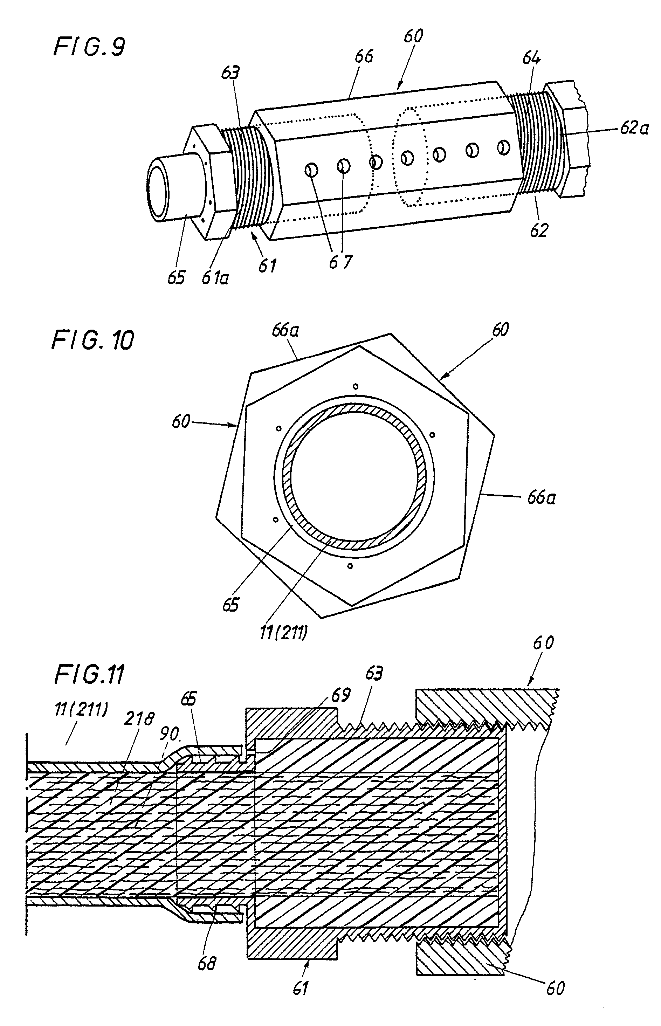

Für das Positionieren und Fixieren von Knochenbruchstücken ist es oftmals erforderlich, den Fixateur externe so auszubilden, daß ein Nachspannen oder überhaupt Spannen möglich ist. Hierzu ist ein Spannelement 60 vorgesehen, das aus zwei auf ihren Außenwandflächen mit Außenwinden versehenen rohrförmigen endseitig verschlossenen Abschnitten 61,62 besteht (Fig. 9). Von den beiden Außengewinden der beiden rohrförmigen Abschnitte 61,62 ist das eine Außengewinde als Rechtsgewinde 63 und das andere Außengewinde als Linksgewinde 64 ausgebildet. Jeder rohrförmige Abschnitt 61,62 weist an seinem äußeren Ende 61a,62a einen Ansatz- bzw. Anschlußstutzen 65 für schlauchförmige Teile 11 auf. Diese Ansatz- bzw. Anschlußstutzen 65 können aus dem Material der rohrförmigen Abschnitte 61, 62 mit ausgebildet sein; es besteht jedoch auch die Möglichkeit, gesonderte Ansatz- bzw. Anschlußstutzen 65 zu verwenden, die deann unter Verwendung geeigneter Verbindungsmittel, wie z.B. Schraubverbindungen, mit den rohrförmigen Abschnitten 61, 62 verbunden sind. Der Außendurchmesser dieser Ansatz- bzw. Anschlußstutzen 65 entspricht dem Innendurchmesser der zu verbindenden schlauchförmigen Teile 11.

Auf die Außengewinde 63, 64 der beiden rohrförmigen Abschnitte 61, 62 ist eine rohrförmige Spannmutter 66 aufgeschraubt, deren Außenwandfläche mit einer Griffprofilierung versehen ist. Bei dem in Fig. 9 gezeigten Ausführungsbeispiel ist die Spannmutter 66 mit einer Anzahl von Griffflächen 66a versehen, die durch eine sechseckige Querschnittsausbildung der Spannmutter erhalten werden (Fig. 10). Diese Spannmutter 66 weist eine Anzahl von Durchbohrungen 67 auf, die in Reihe hintereinander, d.h. nebeneinanderliegend, angeordnet sind und sich in Längsrichtung der Spannmutter erstrecken. Diese Durchbohrungen 67 dienen zur Überwachung und Überprüfung des Spannweges, so daß diese Durchbohrungen 67 die Aufgabe von Beobachtungsfenstern haben. Vorteilhafterweise sind Abschnitte der Außenwandfläche der beiden rohrförmigen Abschnitte 61, 62 des Spannelementes 60 mit einer unterschiedlichen Farbgebung versehen, so daß dann anhand der durch die Durchbohrungen 67 zu erblickenden Farben eine Beurteilung des Spannweges vorgenommen werden kann. Um einen sicheren Sitz des schlauchförmigen Teiles 11 an den Ansatz- bzw. Anschlußstutzen 65 zu erreichen, sind diese an ihrer Außenwandfläche mit einem Wellen- oder Rippenprofil versehen, das in Fig. 11 bei 68 angedeutet ist. Mit 69 ist eine Entlüftungsöffnung bezeichnet. Mit einem derart ausgebildeten Spannelement 60 ist ein Bauelement geschaffen, mit dem ein Spannen der schlauchförmigen Teile z.B. in dem Rahmen 20 mühelos durchführbar ist, so daß neben einem Spannen das Spannelement gleichzeitig überbrückende und zusätzliche Befestigungseigenschaften aufweist. Die rohrförmigen Abschnitte 61,62 können auch als Hohlschrauben ausgebildet sein, deren Innenräume mit Glasfasern gefüllt werden, was durch ein Zurückschneiden des die Glasfaserfüllung aufnehmenden Schlauchkörpers 212 aus Kunststoff erfolgt, so daß ein Abschnitt der reinen Glasfaserfüllung bzw. des Glasfasergewebeschlauches erhalten wird. Dieser Abschnitt der Glasfaserfüllung bzw. des Glasfasergewebeschlauches dient zur Füllung der Hohlschraube und führt somit zu einem festen Verbund zwischen dem Spannelement 60 und dem Fixateurschlauch nach dem Ausgießen mit Kunstharz, welches beim Ausgießen in den freigelegten und sich in dem Innenraum der Hohlschraube befindenden Abschnitt der Glasfaserfüllung fließt. Die Ausbildung des Spannelementes 60 als Sechskant hat gleichzeitig den Vorteil, daß mit entsprechend ausgebildeten Werkzeugen das Spannelement 60 außenseitig ergriffen und um seine Längsachse zum Spannen verdreht werden kann.

Als Fixateurschläuche, d.h. als schlauchförmige Teile 11, können auch Kunststoffschläuche verwendet werden, die außenseitig oder innenseitig mit einem Glasfasergewebe versehen sind, wobei diese Glasfasergewebe bei der Herstellung des Schlauchmaterials aus Kunststoffen in den Kunststoff mit eingelassen werden.

Bei der Anwendung von Kirschner-Drähten 30 ist es von Vorteil, diese Drähte 30 mittels um den Fixateurschlauch und die Drähte 30 gelegte Kabelbinder 80 zu sichern (Fig. 3). Diese Kabelbinder verhindern ein Herausgleiten der Drähte 30 aus ihrer Verankerung im Fixateurschlauch vor und während der Füllung des Fixateurschlauches mit Kunstharz.

Die Fixierungselemente 40,140 und das Spannelement 60 bestehen aus geeigneten Kunststoffen von großer Festigkeit, insbesondere Bruchfestigkeit; jedoch auch andere geeignete Werkstoffe können zur Anwendung gelangen.

Nach Fig. 12 und 13 besteht der schlauchförmige Teil 211 aus einem Schlauchkörper 212 aus einem flexiblen Kunststoff. In den Innenraum des Schlauchkörpers 212 ist ein Schlauch 215 aus einem Glasfasergewebe eingezogen, der an der Innenwand des Schlauchkörpers 212 anliegt. Wird ein Schlauchsystem aus mehreren schlauchförmigen Teilen 211 verwendet, dann werden die Glasfasergewebe-Schläuche in die einzelnen Schlauchabschnitte vor dem Aneinandersetzen und dem Miteinanderverbinden der Schlauchabschnitte eingezogen. Auch länger bemessene Schlauchkörper 212 mit eingezogenem Glasfasergewebeschlauch 215 können verwendet werden und für den Gebrauch auf die erforderlichen Längen geschnitten werden.

In den Innenraum des Schlauchkörpers 212 kann ein Glasfasergewebe-Schlauch 215 eingezogen sein (Fig. 12), jedoch auch mehrere Glasfasergewebe-Schläuche 215,216,217 können unter Ausbildung mehrerer Schichten eingezogen sein (Fig. 13). Den Erfordernissen entsprechend können die Glasfasergewebe-Schläuche verschiedene Wandstärken aufweisen. Ein so ausgebildeter schlauchförmiger Teil 211 wird dann mit dem aushärtbaren Kunststoff gefüllt. In Fig. 14 ist der ausgehärtete Kunststoff bei 90 angedeutet. Der Vorteil besteht darin, daß ein derartiger Fixateurschlauch vor der Anwendung steril gemacht werden kann. Auch der Glasfasergewebe-Schlauch kann vor dem Einziehen in den Schlauchkörper sterilisiert werden. Der schlauchförmige Teil 211 hat somit die Funktion einer Gußform. Der aushärtbare Kunststoff dringt nach erfolgter Eingabe in das Schlauchsystem in das Glasfasergewebe des Glasfasergewebe-Schlauches ein und durchströmt die Glasfasern, so daß es nach dem Aushärten zu einem festen, innigen Verbund zwischen dem Glasfasergewebe und dem Kunststoff kommt. Der äußere Schlauchkörper aus Kunststoff hat lediglich die Funktion des Zusammenhaltens und dient zur äußeren Begrenzung und kann daher dünnwandig ausgebildet sein. Es wird auf diese Weise ein Verbundwerkstoff von hoher Stabilität erhalten. Diese Stabilität ist durch die Verwendung einer verschiedenen Anzahl von Glasfasergewebe-Schlauch-Schichten einstellbar.

Der Schlauchkörper 212 ist nach Fig. 15 und 16 spiralförmig ausgebildet und besteht vorzugsweise aus einem transparenten oder glasklaren Kunststoff. Der Innenraum des Schlauchkörpers 212 ist mit einer Glasfaserfüllung 218 versehen, die aus einer gewickelten Glasfasergewebematte oder mehreren ineinander eingezogenen Glasfasergewebe-Schläuchen besteht. Diese Glasfaserfüllung 218 füllt den Innenraum des Schlauchkörpers 212 aus.

## Ansprüche

1. Fixateur externe (10) aus mindestens einem flexiblen schlauchförmigen Teil (11,211) aus Kunststoff, der außerhalb des Patienten gegenüber den zu fixierenden Knochenfragmenten (K1,K2) anordbar und der mit in den verschiedenen Knochenfragmenten befestigten Drähten (12), Nägeln oder Schrauben verbindbar ist, wobei der mit einem aushärtbaren Material gefüllte schlauchförmige Teil nach dem Aushärten des Materials mit den Drähten, Nägeln oder Schrauben ein festes Gestell bildet, das die Knochenfragmente zum Zusammen-wachsen in einer gewünschten Stellung zusammenhält, dadurch gekennzeichnet, daß der schlauchförmige Teil (11,211) des Fixateur externe (10) aus einem mit Glasfasern oder anderen mineralischen Fasern gefüllten Schlauchkörper (212) besteht, der als Spiralschlauch ausgebildet ist, und daß nach dem Abschluß der Montage in den Innenraum des geschlossenen Schlauchkörpers als aushärtbares Material ein schnellhärten-der Kunststoff eingebbar ist.

2. Fixateur externe (10) aus mindestens einem flexiblen schlauchförmigen Teil (11,211) aus Kunststoff, der außerhalb des Patienten gegenüber den zu fixierenden Knochenfragmenten (K1,K2) anordbar und der mit in den verschiedenen Knochenfragmenten befestigten Drähten (12), Nägeln oder Schrauben verbindbar ist, wobei der mit einem aushärtbaren Material gefüllte schlauchförmige Teil nach dem Aushärten des Materials mit den Drähten, Nägeln oder Schrauben ein festes Gestell bildet, das die Knochenfragmente zum Zusammenwachsen in einer gewünschten Stellung zusammenhält, dadurch gekennzeichnet, daß der Schlauchkörper (212) des Fixateur externe (10) aus einem glasfaserverstärkten Kunststoff besteht und als Spiralschlauch ausgebildet ist, und daß nach dem Abschluß der Montage in den Innenraum des geschlossenen Schlauchkörpers als aushärtbares Material ein schnellhärtender Kunststoff eingebbar ist.

3. Fixateur externe nach Anspruch 2, dadurch gekennzeichnet, daß der glasfaserverstärkte Kunststoff ein in den Kunststoff eingebettetes Glasfasergewebe aufweist.

4. Fixateur externe nach einem der Ansprüche 1 bis 3, dadurch gekennzeichnet, daß der Schlauchkörper (212) zum Zwecke der Überwachung der Verteilung des in den Innenraum des Schlauchkörpers (212) eingegebenen schnellhärtenden Kunststoffes aus einem transparenten oder glasklaren Kunststoff besteht.

5. Fixateur externe nach Anspruch 1, dadurch gekennzeichnet, daß die Glasfaserfüllung des Schlauchkörpers (212) aus mindestens einem in den Schlauchkörper (212) eingezogenen Schlauch (215) aus Glasfasergewebe oder einer zu einem Schlauch gewickelten Glasfasermatte besteht.

6. Fixateur externe nach Anspruch 1 bis 5, dadurch gekennzeichnet, daß die beiden freien Enden (11a,11b) eines schlauchförmigen Teiles (11;211) über ein vorzugsweise Y- oder ein T-förmiges Verbindungsstück (16) miteinander verbunden sind, das mit einer verschließbaren Einfüllöffnung (14) für das aushärtbare Material versehen ist.

7. Fixateur externe nach Anspruch 1 bis 6, dadurch gekennzeichnet, daß das schlauchförmige Teil (11;211) in verschiedenen Ebenen und Richtungen über den Knochenfragmenten geführt ist.

8. Fixateur externe nach Anspruch 1 bis 7, dadurch gekennzeichnet, daß mehrere schlauchförmige Teile (11;211) zu einem Rahmen (20) zusammengefügt sind, wobei alle schlauchförmigen Teile (11;211;21,22,23,24, 25) über rohrförmige Verbindungsstücke (16) derart miteinander verbunden sind, daß die Innenräume aller schlauchförmigen Teile (11;211;21,22,23,24,25) miteinander verbunden sind, wobei das so ausgebildete Schlauchsystem mindestens eine verschließbare Einfüllöffnung für das aushärtbare Material aufweist.

9. Fixateur externe nach Anspruch 8, dadurch gekennzeichnet, daß mehrere schlauchförmige Teile (11;211;21, 22,23,24,25) des Schlauchsystems gruppenweise zusammen-gefaßt sind, wobei das von jeder Gruppe gebildete Schlauchsystem mindestens eine verschließbare Einfüll-öffnung für das aushärtbare Material aufweist.

10. Fixateur externe nach Anspruch 1 bis 9 , dadurch gekennzeichnet, daß bei der Verwendung von Fixier-drähten, wie z.B. Kirschner-Drähten (30), jeder Draht mit seinem freien Ende (30a) nach dem Abbiegen des Drahtendes um etwa 180° in den schlauchförmigen Teil (11;211) versenkt und mit diesem rotationsstabil verbunden ist.

11. Fixateur externe nach Anspruch 1 bis 9, dadurch gekennzeichnet, daß bei der Verwendung von Fixier-Schrauben oder -Nägeln diese mittels eines Fixierungselementes (40;140) an dem schlauchförmigen Teil (11;211) befestigt sind.

12. Fixateur externe nach Anspruch 11 dadurch gekennzeich-net, daß das Fixierungselement (40) aus zwei schalenförmigen, den schlauchförmigen Teil (11;211) umgebenden Teilen (41,42) besteht, die sich zu einem rohrförmigen Bauelement (43) ergänzen, wobei jedes schalenförmige Teil (41;42) mindestens an zwei sich gegenüberliegenden Seiten schalenförmig ausgebildete Abschnitte (44,4; 46,47) aufweist, die sich zu zwei miteinander fluchtenden Befestigungs-stutzen (48,49) ergänzen und zur Aufnahme einer Schraube oder eines Nagels (12) dienen, wobei die schalenförmigen Abschnitte (44,45; 46,47) auf ihrer Außenwand ein Außengewinde (50) zur Aufnahme einer überwurfmutter (51) tragen, die einen Quetschring (55) umgreift, der auf das Ende des Befestigungsstutzens (48;49) aufgesetzt ist.

13. Fixateur externe nach Anspruch 12, dadurch gekennzeichnet, daß jeder schalenförmige Teil (41;42) des Fixierungselementes (40) an seiner Innenwand mindestens zwei Fixierungszapfen, -dorne od.dgl. (52) aufweist.

14. Fixateur externe nach Anspruch 11, dadurch gekennzeichnet, daß das Fixierungselement (140) aus einem durch zwei gegenüberliegende und miteinander fluchtende Durchbrechungen (112,113) in der Wand (111) des schlauchförmigen Teiles (11) hindurchgeführten, mit einem Außengewinde (160) versehenen Rohr (141) besteht, das in seinem Innenraum einen Nagel oder Schraube aufnimmt, wobei das Gewinderohr (141) beidseitig mit einem Abschnitt (142,143) aus dem schlauchförmigen Teil (11) herausgeführt ist und auf seinen Abschnitten (142,143) im Bereich deren Enden (142a,143a) je eine in das Außengewinde (160) des Gewinderohres (141) eingreifende Überwurfmutter (144,145) trägt, die einen am Ende eines jeden Abschnittes (142,143) des Gewinderohres (141) vorgesehenen, an der Außenwandfläche des in dem Gewinderohr (141) angeordneten Nagels oder Schraube (12) zur Anlage beim Anziehen der Überwurfmutter (144;145) bringbaren Quetschring (146,147) übergreift, und daß das Gewinderohr (141) auf seinem den Knochenfragmenten (K1,K2) zugekehrten Abschnitt (143) eine gewinde-tragende, auf dem Außengewinde aufsitzende Überwurfklammer (148) mit in die Wand (111) des schlauchförmigen Teiles (11) eingreifenden Zapfen oder Dorn (149) und auf seinem anderen Abschnitt (142) eine gewindefreie Überwurfklammer (150) mit in die Wand (111) des schlauchförmigen Teils (11;211) eingreifenden Zapfen, Dorn od.dgl. (151) trägt, wobei die gewindefreie Überwurfklammer (150) auf dem Gewinderohr (141) mittels einer Mutter (152) gehalten ist und mit dem Gewinderohr (141) rotationsstabil durch einen Nocken (154) verbunden ist.

15. Fixateur externe nach Anspruch 1 bis 14 dadurch gekennzeichnet, daß jeder frakturüberbrückende Schlauchabschnitt (11;211) ein Spannelement (60) aufweist.

16. Fixateur externe nach Anspruch 15 dadurch gekennzeichnet, daß das Spannelement (60) aus zwei auf ihren Außenwandflächen mit Außengewinden verse-henen rohrförmigen Abschnitten (61,62) besteht, wobei von den beiden Außengewinden der beiden Abschnitte (61,62) das eine Außengewinde als Rechtsgewinde (63) und das andere Außengewinde als Linksgewinde (64) ausgebildet ist, daß jeder rohrförmige Abschnitt (61,62) an seinem außenliegenden Ende (61a,62a) mit einem Ansatz- bzw. Anschlußstutzen (65) für schlauchförmige Teile (11;211) versehen ist, und daß auf die Außengewinde (63,64) der beiden rohrförmigen Abschnitte (61,62) eine rohrförmige Spannmutter (66) aufgeschraubt ist, die mit einer Griffprofilierung, z.B. Griffflächen (66a) und mit einer Anzahl von in Reihe hintereinander und in Spannmutterlängsrichtung liegenden Durchbohrungen (67) versehen ist.

17. Fixateur externe nach Anspruch 16, dadurch gekennzeichnet, daß Abschnitte der Außenwandfläche der beiden rohrförmigen Abschnitte (61,62) des Spannelementes (60) unterschiedliche Farben aufweisen.

**18.** Fixateur externe nach Anspruch 15 und 17, dadurch gekennzeichnet, daß in der Wand der rohrförmigen Abschnitte (61,62) des Spannelementes (60) Lüftungsöffnungen (69) vorgesehen sind.

**19.** Fixateur externe nach Anspruch 10, dadurch gekennzeichnet, daß die Kirschner-Drähte (30) mit dem schlauchförmigen Teil (11;211) mittels Kabelbinder (90) an dem schlauchförmigen Teil (11;211) gehalten sind.

**20.** Fixateur externe nach einem der vorangegangenen Ansprüche 1 bis 18, dadurch gekennzeichnet, daß bei Fixation durch Drähte, Nägel oder Stifte die um-gebogenen und in die Wand (111) des schlauchförmigen Teils (11;211) versenkten Drahtenden durch Kabelbinder an dem schlauchförmigen Teil (11;211) gesichert sind.

**21.** Fixateur externe nach einem der vorangegangenen Ansprüche 1 bis 20, dadurch gekennzeichnet, daß der Schlauchkörper (212) mit den in diesen eingezogenen Schläuchen (215) aus einem Glasfasergewebe mit dem Glasfasergewebe-Schlauch oder den GlasfasergewebeSchläuchen (215,216 ,217) und dem im Innenraum des Schlauchkörpers (212) ausgehärteten Kunststoff ein Verbundelement bildet.

**22.** Fixateur externe nach Anspruch 21, dadurch gekenn-zeichnet, daß die im Innenraum des Schlauchkörpers (212) angeordneten Glasfasergewebe-Schläuche (215, 216,217) gleiche oder unterschiedliche Stärken aufweisen.

**23.** Fixateur externe nach Anspruch 21 und 22, dadurch gekennzeichnet, daß in dem Innenraum des Schlauchkörpers (212) eine Füllung (218) aus mehreren ineinander eingezogenen Glasfasergewebe-Schläuchen oder aus einer zu einem Schlauch gewickelten Glas-fasergewebematte besteht.

**24.** Fixateur externe nach einem der vorangegangenen Ansprüche 1 bis 23, dadurch gekennzeichnet, daß die aushärtbare Kunststoffmasse mit einer Einfärbung versehen ist.

## Claims

**1.** External fixation apparatus (10) comprising at least one flexible hose-like portion (11,211) of plastic which is disposable external to the patient opposite the bone fragments (K1,K2) to be fixed and which is connectable to the wires (12), nails or screws fastened in the various bone fragments, in which the hose-like portion filled with a curable material, subsequent to the material having become cured, forms a solid frame with the wires, nails or screws which holds the bone fragments together for healing in a desired position, characterized in that the hose-like portion (11,211) of the external fixation apparatus (10) com-prises a tubular member (212) which is constructed in the form of a spiral tubing and is filled with glass fibers or other mineral fibers and in that, subsequent to the termination of being mounted, a quick-curing plastic is filled into the interior of the sealed tubular member as curable material.

**2.** External fixation apparatus (10) comprising at least one flexible hose-like portion (11,211) of plastic which is disposable external to the patient opposite the bone fragments (K1,K2) to be fixed and which is connectable to the wires (12), nails or screws fastened in the various bone fragments, in which the hose-like portion filled with a curable material, subsequent to the material having become cured, forms a solid frame with the wires, nails or screws which holds the bone fragments together for healing in a desired position, characterized in that the tubular member (212) of the external fixation apparatus (10) consists of a glass fiber reinforced plastic and is constructed in the form of a spiral tubing and in that, subsequent to the termination of being mounted, a quick-curing plastic is filled into the interior of the closed tubular member as curable material.

**3.** External fixation apparatus according to Claim 2, characterized in that the glass fiber reinforced plastic has a glass fiber fabric embedded in the plastic.

**4.** External fixation apparatus according to any of Claims 1 to 3, characterized in that the tubular member (212), for the purpose of monitoring the distribution of the quick-curing plastic filled into the interior of the tubular member (212) consists of a transparent or pellucid plastic.

**5.** External fixation apparatus according to Claim 1, characterized in that the glass fiber filling of the tubular member (212) is comprised of at least one glass fiber fabric tubing (215) inserted into the tubular member (212) or of a glass fiber mat wound so as to form a hose.

**6.** External fixation apparatus according to Claims 1 to 5, characterized in that the two free ands (11a,11b) of a hose-like portion (11;211) are interconnected by means of a preferably Y-shaped or T-shaped connecting piece (16) which is provided with a sealable filling aperture (14) for the curable material.

7. External fixation apparatus according to Claims 1 to 6, characterized in that the hose-like portion (11;211) is passed in different planes and directions above the bone fragments.

8. External fixation apparatus according to Claims 1 to 7, characterized in that several hose-like portions (11;211) are joined together so as to form a frame (20), in which all hose-like portions (11;211;21,22,23,24,25) are interconnected by means of tubular connecting pieces (16) in such a way that the interiors of all hose-like portions (11;211;21,22,23,24,25) intercommunicate, while the thusly constructed hose system is provided with at least one sealable filling aperture for the curable material.

9. External fixation apparatus according to Claim 8, characterized in that several hose-like portions (11;211;21,22, 23,24,25) of the hose system are combined so as to form groups, wherein the hose system formed by each group is provided with at least one sealable filling aperture for the curable material.

10. External fixation apparatus according to Claims 1 to 9, characterized in that, when fixation wires, such as e.g. Kirschner wires (30) are employed, each wire is, subsequent to the wire end having been bent aside through approximately 180°, with its free end (30a), embedded in the hoselike portion (11;211) and connected with the latter in a rotationally stable manner.

11. External fixation apparatus according to Claims 1 to 9, characterized in that, when fixation screws or nails are employed, these are secured to the hose-like portion (11; 211) with the aid of a fixation means (40;140).

12. External fixation apparatus according to Claim 11, characterized in that the fixation means (40) is comprised of two shell-shaped parts (41,42) surrounding the hose-like portion (11;211) which complement each other so as to form a tubular structural member (43), while each shell-shaped part (41;42), on at least two oppositely located sides, has shell-like-configured sections (44,45;46,47) which supplement each other so as to form two mutually aligned attachment studs (48,49) and which serve to receive a screw or a nail (12), while the shell-shaped sections (44,45;46,47), on their outer wall, are provided with an external thread (50) for receiving a connection nut (51) which engages around the compression ring (55) which is fitted to the end of the attachment stud (48;49).

13. External fixation apparatus according to Claim 12, characterized in that each shell-shaped part (41;42) of the fi-xation means (40) is provided on its inner wall with at least two fixation lugs, fixation mandrels (52) or the like.

14. External fixation apparatus according to Claim 11, characterized in that the fixation means (140) is comprised of a tube (141) provided with an external thread (160) passed through two oppositely located and mutually aligned perforations (112,113) in the wall (111) of the hose-like portion (11) which accommodates , in its interior, a nail or a screw, in which the threaded tube (141) is on both sides passed out of the hose-like portion (11) with a section (142,143) and, on its section (142,143) within the area of their ends (142a, 143a) , carries one connection nut (144, 145) each engaging into the external thread (160) of the threaded tube (141), said connection nut engaging over a compression ring (146,147) provided on the end of each section (142,143) of the threaded tube (141) which can be brought to bear against the outer wall area of the nail or screw (12) disposed in the threaded tube (141) when the connection nut (144;145) is tightened, and in that the threaded tube (141), on its section (143) facing the bone fragments (K1,K2) carries a threaded coupling clamp (148) seated on the external thread with a lug or mandrel (149) engaging into the wall (111) of the hose-like portion (11) and, on its other section (142), an unthreaded coupling clamp (150) with a lug, mandrel (151) or the like engaging into the wall (111) of the hose-like portion (11;211), while the unthreaded coupling clamp (150) is retained on the threaded tube (141) with the aid of a nut (152) and is connected in a rotation-stable manner to the threaded tube (141) by means of a cam (154).

15. External fixation apparatus according to Claims 1 to 14, characterized in that each fracture-bridging hose-like section (11;211) is provided with a tightening member (60).

16. External fixation apparatus according to Claim 15, characterized in that the tightening member (60) consists of two tubular sections (61,62) provided with external threads on its outer wall areas, in which case, of the two external threads of the two sections (61,62), one of the external threads is constructed as a right-hand thread (63) and the other external thread is constructed as a left-hand thread (64), in that each tubular section (61,62), on its external end (61a, 62a), is provided with an adapter or connection piece (65) for hose-like portions (11;211) and in that, on the external threads (63,64) of the two tubular sections (61,62), a tubular tightening nut (66) is screwed which is provided with a grooved profiling, e.g. gripping surfaces (66a) and with a plurality of drilled holes (67) located in consecutive arrangement and in the longitudinal direction of the tightening nut.

17. External fixation apparatus according to Claim 16, charac-teri zed in that sections of the tubular outer wall area of the two tubular sections (61,62) of the tightening member (60) have different colors.

18. External fixation apparatus according to Claims 15 and 17, characterized in that, in the walls of the tubular sections (61,62) of the tightening member (60), ventilation apertures (69) are provided.

19. External fixation apparatus according to Claim 10, characterized in that the Kirschner wires (30) with the tubular portion (11;211) are retained on the hose-like portion (11;211) with the aid of cable ties (90).

20. External fixation apparatus according to any of the preceding Claims 1 to 18, characterized in that, when the fixa-tion is effected by means of wires, nails or pins, the bent-aside wire ends inserted into the wall (111) of the hose-like portion (11;211) are secured to the hose-like portion (11;211) with the aid of cable ties.

21. External fixation apparatus according to any of the preceding Claims 1 to 20, characterized in that the tubular member (212) with the hoses (215) of a glass fiber fabric inserted therein, forms a composite member with the glass fiber fabric hose or the glass fiber fabric hoses (215, 216,217) and the plastic material cured in the interior of the tubular member (212).

22. External fixation apparatus according to Claim 21, characterized in that the glass fiber fabric hoses (215,216,217) that are disposed in the interior of the tubular member (222) possess uniform or differing thicknesses.

23. External fixation apparatus according to Claims 21 and 22, characterized in that, in the interior of the tubular member (212), a filling (218) comprising several inserted-into-each-other glass fiber fabric hoses or a glass fiber fabric mat wound so as to form a hose.

24. External fixation apparatus according to any of the preceding Claims 1 to 23, characterized in that the curable plastic material is dyed.

**Revendications**

1. Fixateur externe (10) constitué par au moins une pièce tubulaire (11, 211) flexible en matière plastique qui peut être placée en dehors du patient en face des fragments osseux à fixer (K1, K2) et qui peut être reliée aux tiges métalliques (12), vis ou clous fixés dans les différents fragments osseux, la pièce tubulaire remplie d'une matière pouvant durcir formant un bâti fixe avec les tiges métalliques, clous ou vis, après que la matière ait durci, bâti qui maintient les fragments osseux dans une position souhaitée pour qu'ils se soudent, caractérisé en ce que la pièce tubulaire (11, 211) du fixateur externe (10) est constituée par un corps tubulaire (212) rempli de fibres de verre ou d'autres fibres minérales qui est configuré comme un tuyau spiralé et qu'une matière plastique à durcissement rapide peut être remplie comme matière pouvant durcir dans l'espace intérieur du corps tubulaire fermé, une fois le montage terminé.

2. Fixateur externe (10) constitué par au moins une pièce tubulaire (11, 211) flexible en matière plastique qui peut être placée en dehors du patient en face des fragments osseux à fixer (K1, K2) et qui peut être reliée aux tiges métalliques (12), vis ou clous fixés dans les différents fragments osseux, la pièce tubulaire remplie d'une matière pouvant durcir formant un bâti fixe avec les tiges métalliques, clous ou vis, après que la matière ait durci, bâti qui maintient les fragments osseux dans une position souhaitée pour qu'ils se soudent, caractérisé en ce que le corps tubulaire (212) du fixateur externe (10) est constitué par une matière plastique renforcée par fibres de verre et qu'il est formé comme un tuyau spiralé et tuyau spiralé et qu'une matière plastique à durcissement rapide peut être remplie comme matière pouvant durcir dans l'espace intérieur du corps tubulaire fermé, une fois le montage terminé.

3. Fixateur externe selon la revendication 2, caractérisé en ce que la matière plastique renforcée par fibres de verre présente un tissu de fils de verre noyé dans la matière plastique.

4. Fixateur externe selon l'une des revendications 1 à 3, caractérisé en ce que le corps tubulaire (212) est constitué par une matière plastique transparente ou claire comme du verre pour permettre le contrôle de la répartition de la matière plastique à durcissement rapide remplie dans l'espace intérieur du corps tubulaire.

5. Fixateur externe selon la revendication 1, caractérisé en ce que le rembourrage de fibres de verre du corps tubulaire (212) est constitué par au moins un tuyau (215) en tissu de fibres de verre passé dans le corps tubulaire (212) ou par une natte de fibres de verre enroulée en tuyau.

6. Fixateur externe selon les revendications 1 à 5, caratérisé en ce que les deux extrémités libres (11a, 11b) d'une pièce tubulaire (11, 211) sont reliées l'une à l'autre par une pièce de raccord (16), de préférence en Y ou en T, qui est pourvue d'un orifice de remplissage (14) pouvant être fermé pour la matière pouvant durcir.

7. Fixateur externe selon les revendications 1 à 6, caractérisé en ce que la pièce tubulaire (11, 211) est guidée dans plusieurs plans et directions au-dessus des fragments osseux.

8. Fixateur externe selon les revendications 1 à 7, caractérisé en ce que plusieurs pièces tubulaires sont assemblées en un cadre (20), toutes les pièces tubulaires (11; 211 ; 21, 22, 23, 24, 25) étant reliées entre elles par des pièces de raccord (16) tubulaires de manière telle que les espaces intérieurs de toutes les pièces tubulaires (11 ; 211 ; 21, 22, 23, 24, 25) sont reliés les uns aux autres, le système de tuyaux ainsi formé présentant au moins un orifice de remplissage pouvant être fermé pour la matière pouvant durcir.

9. Fixateur externe selon la revendication 8, caractérisé en ce que plusieurs pièces tubulaires (11 ; 211 ; 21, 22, 23, 24, 25) du système de tuyaux sont réunies par groupes, le système de tuyaux formé par chaque groupe présentant au moins un orifice de remplissage pouvant être fermé pour la matière pouvant durcir.

10. Fixateur externe selon les revendications 1 à 9, caratérisé en ce que, lors de l'utilisation de broches de fixation, comme par ex. des broches de Kirschner (30), chaque broche est enfoncée avec son extrémité libre (30a) dans la pièce tubulaire (11 ; 211), après courbure de l'extrémité de la broche d'environ 180° et qu'elle est reliée à cette pièce en résistant à la rotation.

11. Fixateur externe selon les revendications 1 à 9, caractérisé en ce que, lors de l'utilisation de vis ou de clous de fixation, ceux-ci sont fixés à la pièce tubulaire (11 ; 211) au moyen d'un élément de fixation (40 ; 140).

12. Fixateur externe selon la revendication 11, caractérisé en ce que l'élément de fixation (40) est constitué par deux parties (41, 42) en forme de coque qui entourent la pièce tubulaire (11 ; 211) et qui se complètent pour former un élément de construction (43) tubulaire, chaque partie en forme de coque (41 ; 42) présentant des portions (44, 45; 46, 47) configurées en forme de coque au moins sur deux côtés opposés l'un à l'autre, portions qui se complètent pour former deux tubulures de fixation (48, 49) alignées l'une sur l'autre et qui servent au logement d'une vis ou d'un clou (12), les portions en forme de coque (44, 45 ; 46, 47) portant, sur leur paroi extérieure, un filet extérieur (50) pour loger un écrouraccord (51) qui enveloppe un anneau comprimé (55) qui est mis sur l'extrémité de la tubulure de fixation (48 ; 49).

13. Fixateur externe selon la revendication 12, caractérisé en ce que chaque partie en forme de coque (41 ; 42) de l'élément de fixation (40) présente, sur sa face intérieure, au moins deux tourillons de fixation, broches de fixation ou équivalent (52).

14. Fixateur externe selon la revendication 11, caractérisé en ce que l'élément de fixation (140) est constitué par un tube (141), pourvu d'un filet extérieur (160), qui traverse deux découpures (112, 113) opposées et alignées l'une sur l'autre qui sont ménagées dans la paroi (111) de la pièce tubulaire (11), tube qui loge, dans son espace intérieur, un clou ou une vis, le tube fileté (141) sortant de la pièce tubulaire (11) des deux côtés avec un tronçon (142, 143) et portant respectivement un écrou-raccord (144, 145) qui s'engrène dans le filet extérieur (160) du tube fileté (141) sur chacun de ses tronçons (142, 143), dans la zone de leurs extrémités (142a, 143a), écrou-raccord qui enveloppe un anneau comprimé (146, 147) prévu à l'extrémité de chacun des tronçons (142, 143) du tube fileté (141) qui peut, lorsque l'on serre l'écrou-raccord (144, 145), être amené contre la surface de la paroi extérieure du clou ou de la vis (12) placée dans le tube fileté (141) et que le tube fileté (141) porte, sur son tronçon (143) tourné vers les fragments osseux (K1, K2), une bride de raccord (148), qui porte un filet et qui est mise sur le filet extérieur, avec un tourillon ou une broche (149) qui s'engrène dans la paroi (111) de la pièce tubulaire (11) et, sur son autre tronçon (12), une bride de raccord (150) sans filet avec un tourillon, une broche ou équivalent (151) qui s'engrène dans la paroi (111) de la pièce tubulaire (11, 211), la bride de raccord sans filet (150) étant maintenue sur le tube fileté (141) au moyen d'un écrou (152) et étant reliée au tube fileté (141) par un ergot (154) de manière à résister à la rotation.

15. Fixateur externe selon les revendications 1 à 14, caractérisé en ce que chaque tronçon de tuyau (11 ; 211) qui surmonte une fracture présente un élément de serrage (60).

16. Fixateur externe selon la revendication 15, caractérisé en ce que l'élément de serrage (60) est constitué par deux tronçons tubulaires (61, 62) pourvus, sur leurs surfaces de paroi extérieure, de filets extérieurs, l'un des deux filets extérieurs des deux tronçons (61, 62) étant formé comme filet droit (63) et l'autre filet extérieur comme filet gauche (64), que chaque tronçon tubulaire (61, 62) est pourvu, à son extrémité située vers l'extérieur (61a, 62a), d'une tubulure à épaulement et/ou de raccord (65) pour les pièces tubulaires (11 ; 211) et qu'un écrou de tension (66) tubulaire est vissé sur les filets extérieurs (63, 64) des deux tronçons tubulaires (61, 62), écrou de tension qui est pourvu d'un profilage de prise, par exemple de surfaces de prise (66a) et d'un certain nombre de perçages (67) alignés les uns derrière les autres et situés dans le sens longitudinal de l'écrou de tension.

17. Fixateur externe selon la revendication 16, caractérisé en ce que des tronçons de la surface de la paroi extérieure des deux tronçons tubulaires (61, 62) de l'élément de serrage (60) présentent différentes couleurs.

18. Fixateur externe selon les revendications 15 et 17, caractérisé en ce que des orifices d'aération (69) sont prévus dans la paroi des portions tubulaires (61, 62) de l'élément de serrage (60).

19. Fixateur externe selon la revendication 10, caractérisé en ce que les broches de Kirschner (30) avec la pièce tubulaire (11 ; 211) sont maintenues au moyen de raccords de câbles (90) sur la pièce tubulaire (11 ; 211).

20. Fixateur externe selon l'une des revendications précédentes 1 à 18, caractérisé en ce qu, lors de la fixation par des tiges métalliques, clous ou chevilles, les extrémités recourbées des tiges métalliques qui sont enfoncées dans la paroi (11) de la pièce tubulaire (11 ; 211) sont fixées à la pièce tubulaire par des raccords de câbles.

21. Fixateur externe selon l'une des revendications précédentes 1 à 20, caractérisé en ce que le corps tubulaire (212) avec les tuyaux (215) en tissu de fibres de verre qui sont passés dans celui-ci forment un élément composite avec le tuyau en tissu de fibres de verre ou les tuyaux en tissu de fibres de verre (215, 216, 217) et la matière plastique durcie dans l'espace intérieur du corps tubulaire (212).

22. Fixateur externe selon la revendication 21, caractérisé en ce que les tuyaux en tissu de fibres de verre (215, 216, 217) placés dans l'espace intérieur du corps tubulaire (212) sont de même épaisseur ou d'épaisseurs différentes.

23. Fixateur externe selon les revendications 21 et 22, caractérisé en ce que, dans l'espace intérieur du corps tubulaire (212), un rembourrage (218) est constitué par plusieurs tuyaux en tissu de fibres de verre passés les uns dans les autres ou par une natte de fibres de verre enroulée en tuyau.

24. Fixateur externe selon l'une des revendications précédentes 1 à 23, caractérisé en ce que la masse de matière plastique pouvant durcir est teintée.

FIG.1

FIG.2

FIG.3

FIG.4

FIG.5

EP 0 260 484 B1

FIG.6

147
142a
150
113a
113
11 (211)
111
143
143a

12
x2
145
140
142
152
151
149
148
160
141
144
146
x3

VII
VII

FIG.7

141
12
112
150
154

FIG.8

156
155

22

FIG.9

FIG. 10

FIG.11

FIG.12

FIG.13

FIG.14

FIG.15

FIG.16

FIG. 17

FIG. 18